# EUROPEAN PATENT SPECIFICATION

(11) **EP 4 142 695 B1**
(45) Date of publication and mention of the grant of the patent: **05.11.2025**
(21) Application number: 21721542.5
(22) Date of filing: 28.04.2021
(51) Int. Cl.: A61K 9/16, A61K 9/20, A61K 38/26, A61P 3/04, A61P 3/10

(54) **SOLID COMPOSITIONS COMPRISING A GLP-1 AGONIST AND HISTIDINE**
FESTE ZUSAMMENSETZUNGEN MIT EINEM GLP-1-AGONISTEN UND HISTIDIN
COMPOSITIONS SOLIDES COMPORTANT UN AGONISTE GLP-1 ET DE L'HISTIDINE

(30) Priority: 29.04.2020 EP 20172111
(43) Date of publication of application: 08.03.2023
(73) Proprietor: NOVO NORDISK A/S, 2880 Bagsværd (DK)
(72) Inventor: JENSEN, Søren, Skov, 2880 Bagsværd (DK); DANCER, Robert, James, 2880 Bagsværd (DK); THORSTHOLM, Louise, 2880 Bagsværd (DK); HACH, Morten, 2880 Bagsværd (DK)
(86) International application number: PCT/EP2021/061125
(87) International publication number: WO 2021/219710

(56) References cited:
- WO-A1-2019/149880
- WO-A1-2019/215063
- US-A1- 2015 072 926
- US-A1- 2019 314 283

## Description

### TECHNICAL FIELD OF THE INVENTION

The present invention relates to solid pharmaceutical compositions comprising a GLP-1 agonist and histidine, their method of preparation and their use in medicine.

### INCORPORATION-BY-REFERENCE OF THE SEQUENCE LISTING

The Sequence Listing, entitled "SEQUENCE LISTING", is 4 KB and was created April 22^{th}, 2021 and is incorporated herein by reference.

### BACKGROUND

Human GLP-1 and analogues thereof have a low oral bioavailability. Exposure and bioavailability of human GLP-1 and analogues thereof is very low following oral administration. Human GLP-1 and analogues thereof can only reach therapeutically relevant plasma concentration after oral administration if formulated with certain absorption enhancers in a specific amount.

Steinert et al. (Am J Clin Nutr, Oct 2010; 92: 810 - 817) discloses oral administration of a tablet comprising GLP-1(7-36)amide and 150 mg sodium N-(8-(2-hydroxybenzoyl)amino)caprylate (SNAC).

WO 2010/020978 discloses an oral pharmaceutical composition comprising a protein and N-(8-[2-hydroxybenzoyl) amino)caprylate (SNAC). Patent applications disclosing oral dosage forms of GLP-1 analogues containing a salt of N-(8-(2-hydroxybenzoyl)amino)caprylate include WO2012/080471, WO2013/189988, WO2013/139694, WO2013/139695 and WO2014/177683. Further improved formulations have been described in WO2019/149880 and WO2019/215063

Independent of the specific formulation the stability of the GLP-1 analogue is of significant interest to ensure that solid GLP-1 compositions maintain efficacy during storage and further to minimize waste of pharmaceutical compositions by extending the shelf-life of the products. Furthermore, the stability of the GLP-1 analogue is of significant interest as it impacts the storage temperature feasible during the shelf-life of the products, which is preferred to be ambient room temperature for solid oral dosage forms.

### SUMMARY

The present invention relates to a solid composition comprising a GLP-1 agonist, an absorption enhancer or delivery agent and histidine, wherein the GLP-1 agonist is semaglutide and the absorption enhancer is a salt of N-(8-(2-hydroxybenzoyl)amino)caprylic acid (NAC). The invention is set out in the appended claims.

The composition according to the invention comprises balanced amounts of the GLP-1 agonist and histidine.

The inventors have surprisingly found that an increased stability of GLP-1 agonists is observed when compositions are prepared with a relatively small amount of histidine.

An embodiment of the invention relates to a solid pharmaceutical composition comprising:
i) a GLP-1 agonist,
ii) a salt of N-(8-(2-hydroxybenzoyl)amino)caprylic acid (NAC) and
iii) histidine
wherein the moles of histidine are at least equal to half the moles of the GLP-1 agonist.

In an embodiment the solid pharmaceutical composition comprises:
i) 0.5-100 mg Semaglutide,
ii) 50-750 mg salt of N-(8-(2-hydroxybenzoyl)amino)caprylic acid (NAC), such as the sodium salt of NAC (SNAC) and
iii) 0.01-200 mg histidine.

A further aspect of the invention relates to a method for producing a solid pharmaceutical composition as described herein comprising the steps of;
i) admixing a GLP-1 agonist and histidine and
ii) preparing said solid pharmaceutical composition using the product of i).

A further aspect of the invention relates to a method for producing a solid pharmaceutical composition as described herein comprising the steps of;
i) co-processing a GLP-1 agonist and histidine and
ii) preparing said solid pharmaceutical composition using the product of i).

A further aspect relates to the medical use of compositions described herein. An embodiment relates to pharmaceutical use of compositions described herein, such as compositions for oral administration. In a further embodiment the composition is a pharmaceutical composition for use in a method of treating diabetes and/or obesity.

In a further aspect, the invention relates to a method of treating diabetes or obesity comprising administering the composition as defined herein to a patient in need thereof.

### BRIEF DESCRIPTION OF DRAWINGS

Fig. 1. shows the amounts of formaldehyde adducts in the tablets measured after 2,4 and 6 weeks.
Fig. 2. shows the amounts of acetaldehyde adducts in the tablets measured after 2,4 and 6 weeks.

### DESCRIPTION

Aspects of the invention described herein relate to solid compositions comprising a GLP-1 agonist and a stability enhancer such as histidine, such as compositions comprising a GLP-1 agonist and an absorption enhancer or delivery agent and histidine. The composition may preferably be in a form suitable for oral administration, such as in a solid form exemplified by a tablet, sachet or capsule. In an embodiment the composition is a composition for oral administration or a pharmaceutical composition, such as an oral pharmaceutical composition.

### GLP-1 agonist

The term "GLP-1 agonist" as used herein refers to a compound, which fully or partially activates the human GLP-1 receptor. The term is thus equal to the term "GLP-1 receptor agonist" used in other documents. The term GLP-1 agonist as well as the specific GLP-1 agonists described herein also encompass salt forms thereof.

It follows that the GLP-1 agonist should display "GLP-1 activity" which refers to the ability of the compound, i.e. a GLP-1 analogue or a compound comprising a GLP-1 analogue, to bind to the GLP-1 receptor and initiate a signal transduction pathway resulting in insulinotropic action or other physiological effects as is known in the art. In some embodiments the "GLP-1 agonist" binds to a GLP-1 receptor, e.g., with an affinity constant (K_{D}) or activate the receptor with a potency (EC₅₀) of below 1 µM, e.g. below 100 nM as measured by methods known in the art (see e.g. WO 98/08871) and exhibits insulinotropic activity, where insulinotropic activity may be measured *in vivo* or *in vitro* assays known to those of ordinary skill in the art. For example, the GLP-1 agonist may be administered to an animal with increased blood glucose (e.g. obtained using an Intravenous Glucose Tolerance Test (IVGTT)). A person skilled in the art will be able to determine a suitable glucose dosage and a suitable blood sampling regime, e.g. depending on the species of the animal, for the IVGTT and measure the plasma insulin concentration over time. Suitable assays have been described in such as WO2015/155151.

The term half maximal effective concentration (EC₅₀) generally refers to the concentration which induces a response halfway between the baseline and maximum, by reference to the dose response curve. EC₅₀ is used as a measure of the potency of a compound and represents the concentration where 50% of its maximal effect is observed. Due to the albumin binding effects of GLP-1 agonists comprising a substituent as described herein, it is important to pay attention to if the assay includes human serum albumin or not.

The in vitro potency of the GLP-1 agonist may be determined as described in 2015/155151, example 29 without Human Serum Albumin (HSA), and the EC₅₀ determined. The lower the EC₅₀ value, the better the potency. In one embodiment the potency (EC50) as determined (without HSA) is 5-1000 pM, such as 10-750 pM, 10-500 pM or 10-200 pM. In one embodiment the EC50 (without HSA) is at most 500 pM, such as at most 300 pM, such as at most 200 pM.

In one embodiment the EC50 (without HSA) is comparable to human GLP-1(7-37).

In one embodiment the EC50 (without HSA) is at most 50 pM. In a further such embodiment the EC50 is at most 40 pM, such as at most 30 pM such as at most 20 pM, such as at most 10 pM. In one embodiment the EC50 is around 10 pM.

Also, or alternatively, the binding of the GLP-1 agonist to albumin may be measured using the in vitro potency assay of Example 29 including HSA. An increase of the in vitro potency, EC₅₀ value, in the presence of serum albumin reflects the affinity to serum albumin.

In one embodiment the potency (EC50) as determined (with 1 % HSA) is 5-1000 pM, such as 100-750 pM, 200-500 pM or 100-400 pM. In one embodiment the EC50 (with 1 % HSA) is at most 750 pM, such as at most 500 pM, such as at most 400 pM, such as at most 300 or such as at most 250 pM.

If desired, the fold variation in relation to a known GLP-1 receptor agonist may be calculated as EC50(test analogue)/EC50(known analogue), and if this ratio is such as 0.5-1.5, or 0.8-1.2 the potencies are considered to be equivalent.

In one embodiment the potency, EC50 (without HSA), is equivalent to the potency of liraglutide.

In one embodiment the potency, EC50 (without HSA), is equivalent to the potency of semaglutide.

In one embodiment the potency, EC50 (without HSA), is equivalent to the potency of GLP-1 agonist B.

In one embodiment the potency, EC50 (without HSA), is equivalent to the potency of GLP-1 agonist C.

In one embodiment the potency, EC50 (with 1 % HSA), is equivalent to the potency of liraglutide.

In one embodiment the potency, EC50 (with 1 % HSA), is equivalent to the potency of semaglutide.

In one embodiment the potency, EC50 (with 1 % HSA), is equivalent to the potency of GLP-1 agonist B.

In one embodiment the potency, EC50 (with 1 % HSA), is equivalent to the potency of GLP-1 agonist C.

In one embodiment a GLP-1 agonist is a bifunctional molecule such as co-agonist, or tri-agonist.

In one embodiment the GLP-1 agonist is also a Gastric inhibitory polypeptide receptor agonist (GIP agonist). In one embodiment the GLP-1 agonist is Tirzepatide.

In some embodiments the GLP-1 agonist is a GLP-1 analogue, optionally comprising one substituent. The term "analogue" as used herein referring to a GLP-1 peptide (hereafter "peptide") means a peptide wherein at least one amino acid residue of the peptide has been substituted with another amino acid residue and/or wherein at least one amino acid residue has been deleted from the peptide and/or wherein at least one amino acid residue has been added to the peptide and/or wherein at least one amino acid residue of the peptide has been modified. Such addition or deletion of amino acid residues may take place at the N-terminal of the peptide and/or at the C-terminal of the peptide. In some embodiments a simple nomenclature is used to describe the GLP-1 agonist, e.g., [Aib8] GLP-1(7-37) designates an analogue of GLP-1(7-37) wherein the naturally occurring Ala in position 8 has been substituted with Aib. In some embodiments the GLP-1 agonist comprises a maximum of twelve, such as a maximum of 10, 8 or 6, amino acids which have been altered, e.g., by substitution, deletion, insertion and/or modification, compared to e.g. GLP-1(7-37). In some embodiments the analogue comprises up to 10 substitutions, deletions, additions and/or insertions, such as up to 9 substitutions, deletions, additions and/or insertions, up to 8 substitutions, deletions, additions and/or insertions, up to 7 substitutions, deletions, additions and/or insertions, up to 6 substitutions, deletions, additions and/or insertions, up to 5 substitutions, deletions, additions and/or insertions, up to 4 substitutions, deletions, additions and/or insertions or up to 3 substitutions, deletions, additions and/or insertions, compared to e.g. GLP-1(7-37). Unless otherwise stated the GLP-1 comprises only L-amino acids.

In some embodiments the term "GLP-1 analogue" or "analogue of GLP-1" as used herein refers to a peptide, or a compound, which is a variant of the human Glucagon-Like Peptide-1 (GLP-1(7-37)). GLP-1(7-37) has the sequence HAEGTFTSDV SSYLEGQAAKEFIAWLVKGRG (SEQ ID No: 1). In some embodiments the term "variant" refers to a compound which comprises one or more amino acid substitutions, deletions, additions and/or insertions.

In one embodiment the GLP-1 agonist exhibits at least 60%, 65%, 70%, 80% or 90% sequence identity to GLP-1(7-37) over the entire length of GLP-1(7-37). As an example of a method for determination of sequence identity between two analogues the two peptides [Aib8]GLP-1(7-37) and GLP-1(7-37) are aligned. The sequence identity of [Aib8]GLP-1(7-37) relative to GLP-1(7-37) is given by the number of aligned identical residues minus the number of different residues divided by the total number of residues in GLP-1(7-37). Accordingly, in said example the sequence identity is (31-1)/31.

In one embodiment the C-terminal of the GLP-1 agonist is an amide.

In some embodiments the GLP-1 agonist is GLP-1(7-37) or GLP-1(7-36)amide. In some embodiments the GLP-1 agonist is exendin-4, the sequence of which is HGEGTFITSDLSKQMEEEAVRLFIEWLKNGGPSSGAPPPS (SEQ ID No: 2). In one embodiment the GLP-1 agonist is an exendin-4 analogue or an engineered peptide thereof, as disclosed in WO2013009545 and references therein.

In order to prolong the effect of the GLP-1 agonist it is preferred that the GLP-1 agonist have an extended half-life. The half-life can be determined by method known in the art an in an appropriate model, such as in Male Sprague Dawley rats or minipigs as described in WO2012/140117. Half-life in rats may be determined as in Example 39 and the half-life in minipigs may be determined as in Example 37 therein.

In one embodiment the GLP-1 agonist according to the invention has a half-life above 2 hours in rat. In one embodiment the GLP-1 agonist according to the invention has a half-life above 4 hours, such as above 6 hours, such as above 8 hours, such as above 10 hours, such as above 12 hours or such as above 15 hours in rat.

In one embodiment the GLP-1 agonist according to the invention has a half-life above 24 hours in minipig. In one embodiment the GLP-1 agonist according to the invention has a half-life above 30 hours, such as above 36 hours, such as above 42 hours, such as above 48 hours, such as above 54 hours or such as above 60 hours in minipig.

In one embodiment the GLP-1 agonist has a molecular weight of at most 50 000 Da, such as at most 40 000 Da, such as at most 30 000 Da.

In one embodiment the GLP-1 agonist has a molecular weight of at most 20 000, such as at most 10 000 Da, such as at most 7 500 Da, such as at most 5 000 Da.

In one embodiment the GLP-1 agonist has a molar mass of at most 50 000 g/mol, such as at most 40 000 g/mol, such as at most 30 000 g/mol.

In one embodiment the GLP-1 agonist has a molar mass of at most 10 000 g/mol, such as at most 8 000 g/mol, such as at most 6 000 g/mol.

In some embodiments the GLP-1 agonist comprises one substituent which is covalently attached to the peptide. In some embodiments the substituent comprises a fatty acid or a fatty diacid. In some embodiments the substituent comprises a C16, C18 or C20 fatty acid. In some embodiments the substituent comprises a C16, C18 or C20 fatty diacid.

In some embodiments the substituent comprises formula (X) 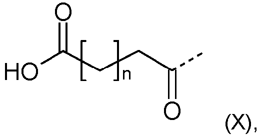 wherein n is at least 13, such as n is 13, 14, 15, 16, 17, 18 or 19. In some embodiments the substituent comprises formula (X), wherein n is in the range of 13 to 19, such as in the range of 13 to 17. In some embodiments the substituent comprises formula (X), wherein n is 13, 15 or 17. In some embodiments the substituent comprises formula (X), wherein n is 13. In some embodiments the substituent comprises formula (X), wherein n is 15. In some embodiments the substituent comprises formula (X), wherein n is 17.

In some embodiments the substituent comprises formula (Xla)
HOOC-(C₆H₄)-O-(CH₂)ₘ-CO-* (Xla), wherein m is an integer in the range of 6-14

In some embodiments the substituent comprises formula (Xlb) 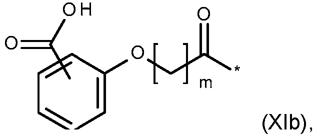 wherein the carboxy group is in position 2, 3 or 4 of the (C₆H₄) group and wherein m is an integer in the range of 8-11.

In some embodiments the substituent comprises formula (Xla) or formula (XIb), wherein m is in the range of 6 to 14, such as in the range of 8 to 11. In some embodiments the substituent comprises formula (Xla) or formula (Xlb), wherein m is 8, 10 or 12. In some embodiments the substituent comprises formula (Xla) or formula (Xlb), wherein m is 9. In some embodiments the substituent comprises formula (Xla) or formula (Xlb), wherein m is 11.

In some embodiments the substituent comprises one or more 8-amino-3,6-dioxaoctanoic acid (OEG), such as two OEG.

In some embodiments the substituent is [2-(2-{2-[2-(2-{2-[(S)-4-carboxy-4-(17-carboxyheptadecanoylamino) butyrylamino]ethoxy}ethoxy)acetylamino] ethoxy}ethoxy)acetyl].

In some embodiments the substituent is [2-(2-{2-[2-(2-{2-[(S)-4-carboxy-4-({trans-4-[(19-carboxynonadecanoylamino)methyl]cyclohexanecarbonyl} amino)butyrylamino]ethoxy}ethoxy)acetylamino]ethoxy}ethoxy)acetyl].

In some embodiments the GLP-1 agonist is semaglutide, also known as N-epsilon26-[2-(2-{2-[2-(2-{2-[(S)-4-carboxy-4-(17-carboxyheptadecanoylamino) butyrylamino]ethoxy}ethoxy)acetylamino]ethoxy}ethoxy)acetyl][Aib8,Arg34]GLP-1(7-37), (SEQ ID NO. 4) which may be prepared as described in WO2006/097537, Example 4 with the following structure:

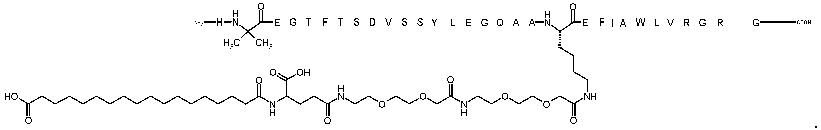

In one embodiment the GLP-1 agonist is GLP-1 agonist B, which is diacylated [Aib8,Arg34,Lys37]GLP-1 (7-37) (SEQ ID NO. 5) as shown in Example 2 of WO2011/080103 and named *N^{ε26}*{2-[2-(2-{2-[2-(2-{(S)-4-Carboxy-4-[10-(4-carboxyphenoxy)decanoylamino]butyrylamino}-ethoxy)ethoxy]acetylamino}ethoxy)ethoxy]acetyl}, *N^{ε37}*-{2-[2-(2-{2-[2-(2-{(S)-4-carboxy-4-[10-(4-carboxyphenoxy)decanoylamino]butyrylamino}ethoxy)ethoxy]acetylamino}ethoxy)ethoxy]-acetyl}-[Aib⁸,Arg³⁴,Lys³⁷]GLP-1 (7-37)-peptide with the following structure.

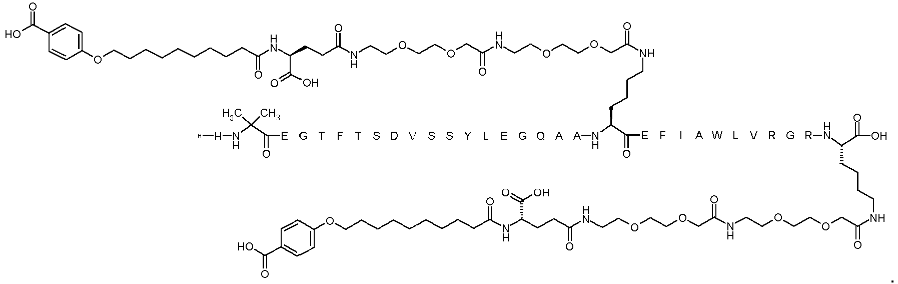

In one embodiment the GLP-1 agonist is GLP-1 agonist C which is Diacylated [Aib8,Glu22,Arg26,Lys27,Glu30,Arg34,Lys36]-GLP-1-(7-37)-peptidyl-Glu-Gly (SEQ ID NO. 6) as shown in Example 31 of WO2012/140117 and named N^{ε27}-[2-[2-[2-[[2-[2-[2-[[(4S)-4-carboxy-4-[10-(4-carboxyphenoxy)decanoylamino]butanoyl]amino] ethoxy]ethoxy]acetyl]amino]ethoxy]ethoxy]-acetyl], N^{ε36}-[2-[2-[2-[[2-[2-[2-[[(4S)-4-carboxy-4-[10-(4-carboxyphenoxy)decanoylamino]-butanoyl]amino]ethoxy]ethoxy]acetyl]amino]ethoxy]ethoxy]acetyl]-[Aib8,Glu22,Arg26,Lys27, Glu30,Arg34,Lys36]-GLP-1-(7-37)-peptidyl-Glu-Gly with the following structure:

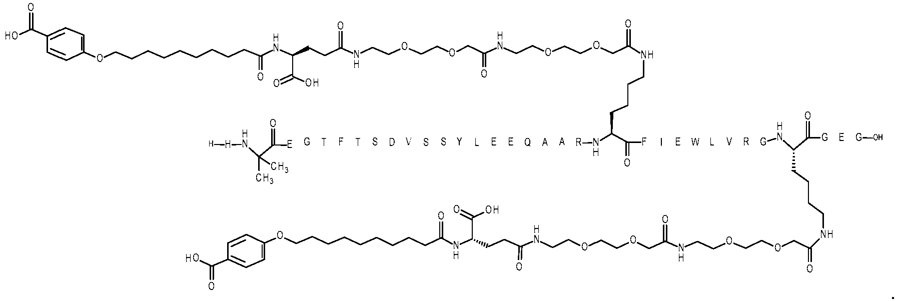

In general, the term GLP-1 agonist is meant to encompass the GLP-1 agonist and any pharmaceutically acceptable salt, amide, or ester thereof. In some embodiments the composition comprises the GLP-1 agonist or a pharmaceutically acceptable salt, amide, or ester thereof. In some embodiments the composition comprises the GLP-1 agonist and one or more pharmaceutically acceptable counter ions.

In some embodiments the GLP-1 agonist is selected from one or more of the GLP-1 agonists mentioned in WO93/19175, WO96/29342, WO98/08871, WO99/43707, WO99/43706, WO99/43341, WO99/43708, WO2005/027978, WO2005/058954, WO2005/058958, WO2006/005667, WO2006/037810, WO2006/037811, WO2006/097537, WO2006/097538, WO2008/023050, WO2009/030738, WO2009/030771 and WO2009/030774.

In some embodiments the GLP-1 agonist is selected from the group consisting of N-epsilon37{2-[2-(2-{2-[2-((R)-3-carboxy-3-{[1-(19-carboxynonadecanoyl) piperidine-4-carbonyl]amino}propionylamino)ethoxy]ethoxy}acetylamino)ethoxy]ethoxy}acetyl [desaminoHis7,Glu22,Arg26,Arg34,Lys37]GLP-1(7-37)amide; N-epsilon26{2-[2-(2-{2-[2-((R)-3-carboxy-3-{[1-(19-carboxynonadecanoyl) piperidine-4-carbonyl]amino} propionylamino)ethoxy]ethoxy}acetylamino)ethoxy] ethoxy}acetyl [desaminoHis7, Arg34] GLP-1-(7-37); N-epsilon37{2-[2-(2-{2-[2-((S)-3-carboxy-3-{[1-(19-carboxy-nonadecanoyl) piperidine-4-carbonyl]amino}propionylamino)ethoxy] ethoxy} acetylamino)ethoxy] ethoxy}acetyl[Aib8,Glu22,Arg26,Arg34,Lys37]GLP-1-(7-37)amide; N-epsilon37-[2-(2-[2-(2-[2-(2-((R)-3-[1-(17-carboxyheptadecanoyl)piperidin-4-ylcarbonylamino]3-carboxypropionylamino)ethoxy)ethoxy]acetylamino)ethoxy] ethoxy)acetyl][,DesaminoHis7, Glu22 Arg26, Arg 34, Phe(m-CF3)28]GLP-1-(7-37)amide; N-epsilon26-[(S)-4-carboxy-4-({trans-4-[(19-carboxynonadecanoylamino)methyl] cyclohexanecarbonyllamino)butyryl][Aib8,Arg34]GLP-1-(7-37); N-epsilon26-{4-[(S)-4-carboxy-4-({trans-4-[(19-carboxynonadecanoylamino) methyl]cyclohexanecarbonyl} amino)butyrylamino]butyryl}[Aib8,Arg34]GLP-1-(7-37); N-epsilon26-[2-(2-{2-[(S)-4-carboxy-4-({trans-4-[(19-carboxy-nonadecanoylamino) methyl]cyclohexanecarbonyl} amino)butyrylamino]ethoxy}ethoxy)acetyl][Aib8,Arg34]GLP-1-(7-37); N-epsilon26-[2-(2-{2-[2-(2-{2-[(S)-4-carboxy-4-({trans-4-[(19-carboxy-nonadecanoylamino)methyl] cyclohexanecarbonyl}amino)butyrylamino]ethoxy}ethoxy)acetylamino]ethoxy}ethoxy) acetyl][Aib8,Arg34]GLP-1-(7-37)amide; N-epsilon37-[2-(2-{2-[2-(2-{2-[(S)-4-carboxy-4-({trans-4-[(19-carboxy-nonadecanoylamino)methyl]cyclohexanecarbonyl}amino) butyrylamino]ethoxy}ethoxy)acetylamino]ethoxy}ethoxy)acetyl][Aib8,Glu22,Arg26, Arg34,Lys37]GLP-1-(7-37)amide; N-epsilon37-[2-(2-{2-[2-(2-{2-[(S)-4-carboxy-4-({trans-4-[(19-carboxy-nonadecanoylamino)methyl]cyclohexanecarbonyl}amino) butyrylamino] ethoxy}ethoxy)acetylamino]ethoxy}ethoxy)acetyl][DesaminoHis7,Glu22, Arg26,Arg34,Lys37]GLP-1-(7-37)amide; N-epsilon37-[2-(2-{2-[2-(2-{2-[(S)-4-carboxy-4-({4-[(trans-19-carboxy-nonadecanoylamino)methyl]cyclohexanecarbonyl}amino) butyrylamino]ethoxy}ethoxy)acetylamino]ethoxy}ethoxy)acetyl][DesaminoHis7,Arg26,Arg34,L ys37]GLP-1-(7-37)amide; N-epsilon37-[2-(2-{2-[2-(2-{2-[(S)-4-carboxy-4-({trans-4-[(19-carboxy-nonadecanoylamino)methyl]cyclohexanecarbonyl}amino) butyrylamino]ethoxy}ethoxy)acetylamino]ethoxy}ethoxy)acetyl][DesaminoHis7,Glu22,Arg26,A rg34,Lys37]GLP-1-(7-37); N-epsilon26[2-(2-{2-[2-(2-{2-[(S)-4-carboxy-4-({4-[(19-carboxy-nonadecanoylamino)methyl]cyclohexanecarbonyl}amino)butyrylamino] ethoxy}ethoxy) acetylamino]ethoxy}ethoxy)acetyl[Aib8, Lys 26]GLP-1 (7-37)amide; N-epsilon26 [2-(2-[2-(2-[2-(2-((S)-2-[trans-4-((9-carboxynonadecanoylamino] methyl) cyclohexylcarbonylamino]-4-carboxybutanoylamino)ethoxy)ethoxy]acetylamino) ethoxy]ethoxy)acetyl][Aib8, Lys26] GLP-1 (7-37)amide; N-epsilon37-[2-(2-{2-[2-(2-{2-[(S)-4-carboxy-4-({trans-4-[(19-carboxy-nonadecanoylamino)methyl]cyclohexane-carbonyl} amino)butyrylamino]ethoxy}ethoxy)acetylamino]ethoxy}ethoxy)acetyl] [DesaminoHis7,Arg26,Arg34,Lys37]GLP-1-(7-37); N-epsilon37-[2-(2-{2-[2-(2-{2-[(S)-4-carboxy-4-({trans-4-[(19-carboxy-nonadecanoylamino)methyl]cyclohexanecarbonyl} amino)butyrylamino]ethoxy}ethoxy)acetylamino]ethoxy}ethoxy)acetyl][DesaminoHis7,Glu22, Arg26,Glu30,Arg34,Lys37]GLP-1-(7-37); N-epsilon26-[2-(2-{2-[(S)-4-carboxy-4-((S)-4-carboxy-4-{4-[4-(16-(1H-tetrazol-5-yl)-hexadecanoylsulfamoyl)butyrylamino]-butyrylamino}butyrylamino) butyrylamino] ethoxy}ethoxy)acetyl][Aib8,Arg34]GLP-1-(7-37); N-epsilon26-[2-(2-{2-[(S)-4-carboxy-4-((S)-4-carboxy-4-{12-[4-(16-(1H-tetrazol-5-yl)hexadecanoyl-sulfamoyl)butyrylamino]dodecanoylamino}butyrylamino) butyrylamino]ethoxy}ethoxy)acetyl][Aib8,Arg34]GLP-1-(7-37); N-epsilon26-[2-(2-{2-[(S)-4-carboxy-4-((S)-4-carboxy-4-{6-[4-(16-(1H-tetrazol-5-yl)hexadecanoyl-sulfamoyl)butyrylamino]hexanoylamino} butyrylamino)butyrylamino]ethoxy}ethoxy) acetyl][Aib8,Arg34]GLP-1-(7-37); N-epsilon26-[2-(2-{2-[(S)-4-carboxy-4-((S)-4-carboxy-4-{4-[4-(16-(1H-tetrazol-5-yl)hexadecanoylsulfamoyl)butyrylamino] butyrylamino}butyrylamino)butyrylamino]ethoxy}ethoxy)acetyl][Aib8,Arg34]GLP-1-(7-34); N-epsilon26-[2-(2-{2-[(S)-4-carboxy-4-((S)-4-carboxy-4-{12-[4-(16-(1H-tetrazol-5-yl)hexadecanoylsulfamoyl)butyrylamino]-dodecanoylamino}butyrylamino) butyrylamino] ethoxy}ethoxy)acetyl][Aib8,Arg34]GLP-1-(7-34); N-epsilon26-[2-(2-{2-[(S)-4-carboxy-4-((S)-4-carboxy-4-{6-[4-(16-(1H-tetrazol-5-yl)hexadecanoylsulfamoyl) butyrylamino]hexanoylamino}butyrylamino) butyrylamino]ethoxy}ethoxy)acetyl] [Aib8,Arg34]GLP-1-(7-34); N-epsilon26-[2-(2-{2-[(S)-4-carboxy-4-((S)-4-carboxy-4-{12-[4-(16-(1H-tetrazol-5-yl)hexadecanoyl-sulfamoyl)butyrylamino]dodecanoylamino} butyrylamino)butyrylamino]ethoxy}ethoxy)acetyl][Aib8,Arg34]GLP-1-(7-35); N-epsilon26-[2-(2-{2-[(S)-4-carboxy-4-((S)-4-carboxy-4-{6-[4-(16-(1H-tetrazol-5-yl)hexadecanoylsulfamoyl)butyrylamino]hexanoylamino} butyrylamino)butyrylamino] ethoxy}ethoxy)acetyl][Aib8,Arg34]GLP-1-(7-35); N-epsilon26-[2-(2-{2-[(S)-4-carboxy-4-((S)-4-carboxy-4-{6-[4-(16-(1H-tetrazol-5-yl)hexadecanoylsulfamoyl)butyrylamino] hexanoylamino}butyrylamino)butyrylamino]ethoxy}ethoxy)acetyl][Aib8,Arg34]GLP-1-(7-36)amide; N-epsilon26-[2-(2-{2-[(S)-4-carboxy-4-((S)-4-carboxy-4-{6-[4-(16-(1H-tetrazol-5-yl)hexadecanoylsulfamoyl) butyrylamino]hexanoylamino}butyrylamino) butyrylamino]ethoxy}ethoxy)acetyl][Aib8,Arg34]GLP-1-(7-35); N-epsilon26-[2-(2-{2-[(S)-4-carboxy-4-((S)-4-carboxy-4-{12-[4-(16-(1H-tetrazol-5-yl)hexadecanoyl-sulfamoyl)butyrylamino]dodecanoylamino}butyryl-amino)butyrylamino]ethoxy} ethoxy)acetyl][Aib8,Lys33,Arg34]GLP-1-(7-34); N-epsilon26-[2-(2-{2-[(S)-4-carboxy-4-((S)-4-carboxy-4-{12-[4-(16-(1H-tetrazol-5-yl)hexadecanoylsulfamoyl)butyrylamino] dodecanoylamino}butyrylamino)butyrylamino]ethoxy}ethoxy)acetyl][Aib8,Arg34]GLP-1-(7-36)amide; N-epsilon26-[2-(2-{2-[2-(2-{2-[2-(2-{2-[2-(2-{2-[2-(2-{2-[2-(2-{2-[(S)-4-carboxy-4-((S)-4-carboxy-4-{12-[4-(16-(1H-tetrazol-5-yl)hexadecanoylsulfamoyl) butyrylamino]dodecanoylamino}butyrylamino) butyrylamino]ethoxy}ethoxy) acetylamino]ethoxy}ethoxy)acetylamino]ethoxy}ethoxy)acetylamino]ethoxy}ethoxy)acetylami no]ethoxy}ethoxy)acetylamino]ethoxy}ethoxy)acetyl][Aib8,Lys26,Arg34]GLP-1-(7-36)amide; N-epsilon37-[2-(2-{2-[(S)-4-carboxy-4-((S)-4-carboxy-4-{12-[4-(16-(1H-tetrazol-5-yl)hexadecanoylsulfamoyl)butyrylamino] dodecanoylamino}butyrylamino) butyrylamino]ethoxy}ethoxy)acetyl][Aib8,Glu22,Arg26,Arg34,Lys37]GLP-1-(7-37)amide; N-epsilon37-[2-(2-{2-[(S)-4-carboxy-4-((S)-4-carboxy-4-{12-[4-(16-(1H-tetrazol-5-yl)hexadecanoylsulfamoyl)butyrylamino]dodecanoylamino}butyrylamino) butyrylamino] ethoxy}ethoxy)acetyl][DesaminoHis7,Glu22,Arg26,Arg34,Lys37]GLP-1-(7-37)amide; N-epsilon37{2-[2-(2-{2-[2-((R)-3-carboxy-3-{[1-(19-carboxy-nonadecanoyl) piperidine-4-carbonyl]amino}propionylamino)ethoxy]ethoxy} acetylamino)ethoxy] ethoxy}acetyl [desaminoHis7,Glu22,Arg26,Arg34,Lys37]GLP-1(7-37)amide; N-epsilon37{2-[2-(2-{2-[2-((S)-3-carboxy-3-{[1-(19-carboxynonadecanoyl) piperidine-4-carbonyl]amino} propionylamino) ethoxy]ethoxy}acetylamino)ethoxy] ethoxy} acetyl [Aib8,Glu22, Arg26,Arg34, Lys37]GLP-1-(7-37)amide; N-epsilon37-[2-(2-[2-(2-[2-(2-((R)-3-[1-(17-carboxyhepta-decanoyl)piperidin-4-ylcarbonylamino]3-carboxy-propionylamino) ethoxy)ethoxy] acetylamino) ethoxy] ethoxy)acetyl] [DesaminoHis7, Glu22,Arg26, Arg34,Phe(m-CF3)28] GLP-1-(7-37)amide; N-epsilon37-[2-(2-{2-[2-(2-{2-[(S)-4-carboxy-4-({trans-4-[(19-carboxy-nonadecanoylamino)methyl] cyclohexanecarbonyl} amino)butyrylamino]ethoxy} ethoxy)acetylamino] ethoxy}ethoxy)acetyl] [Aib8,Glu22,Arg26,Arg34,Lys37]GLP-1-(7-37)amide; N-epsilon37-[2-(2-{2-[2-(2-{2-[(S)-4-carboxy-4-({trans-4-[(19-carboxy-nonadecanoylamino)methyl]cyclohexane-carbonyl} amino)butyrylamino]ethoxy}ethoxy) acetylamino]ethoxy}ethoxy)acetyl] [DesaminoHis7,Glu22,Arg26,Arg34,Lys37]GLP-1-(7-37)amide; N-epsilon37-[2-(2-{2-[2-(2-{2-[(S)-4-carboxy-4-({trans-4-[(19-carboxy-nonadecanoylamino)methyl] cyclohexanecarbonyl}amino)butyrylamino]ethoxy}ethoxy) acetylamino]ethoxy} ethoxy)acetyl] [DesaminoHis7,Glu22,Arg26,Arg34, Lys37]GLP-1-(7-37); N-epsilon37-[2-(2-{2-[2-(2-{2-[(S)-4-carboxy-4-({trans-4-[(19-carboxy-nonadecanoylamino) methyl]cyclohexane-carbonyl}amino)butyrylamino]ethoxy}ethoxy) acetylamino] ethoxy}ethoxy)acetyl] [DesaminoHis7,Glu22,Arg26,Glu30,Arg34, Lys37]GLP-1-(7-37); N-epsilon37-[2-(2-{2-[(S)-4-carboxy-4-((S)-4-carboxy-4-{12-[4-(16-(1H-tetrazol-5-yl)hexadecanoyl-sulfamoyl) butyrylamino]dodecanoylamino} butyrylamino) butyrylamino] ethoxy}ethoxy)acetyl] [Aib8,Glu22,Arg26,Arg34,Lys37]GLP-1-(7-37)amide; N-epsilon37-[2-(2-{2-[(S)-4-carboxy-4-((S)-4-carboxy-4-{12-[4-(16-(1H-tetrazol-5-yl)hexadecanoylsulfamoyl) butyrylamino]dodecanoylamino}butyrylamino) butyrylamino] ethoxy}ethoxy)acetyl] [DesaminoHis7,Glu22,Arg26,Arg34,Lys37]GLP-1-(7-37)amide; N-epsilon37-(3-((2-(2-(2-(2-(2-Hexadecyloxyethoxy)ethoxy)ethoxy) ethoxy) ethoxy)) propionyl)[DesaminoHis7,Glu22,Arg26,Arg34,Lys37]GLP-1(7-37)-amide; N-epsilon37-{2-(2-(2-(2-[2-(2-(4-(hexadecanoylamino)-4-carboxybutyryl-amino)ethoxy) ethoxy] acetyl)ethoxy)ethoxy)acetyl)}-[desaminoHis7,Glu22,Arg26, Glu30,Arg34,Lys37] GLP-1-(7-37)amide; N-epsilon37-{2-(2-(2-(2-[2-(2-(4-(hexadecanoylamino)-4-carboxy-butyryl-amino) ethoxy)ethoxy]acetyl)ethoxy)ethoxy) acetyl)}-[desaminoHis7,Glu22, Arg26, Arg34,Lys37]GLP-1-(7-37)amide; N-epsilon37-(2-(2-(2-(2-(2-(2-(2-(2-(2-(octadecanoyl-amino)ethoxy)ethoxy) acetylamino)ethoxy) ethoxy)acetylamino) ethoxy)ethoxy) acetyl)[desaminoHis7,Glu22,Arg26,Arg34,Lys37] GLP-1 (7-37)amide; N-epsilon37-[4-(16-(1H-Tetrazol-5-yl)hexadecanoylsulfamoyl) butyryl] [DesaminoHis7,Glu22,Arg26, Arg34, Lys37]GLP-1-(7-37)amide; N-epsilon37-[2-(2-{2-[2-(2-{2-[(S)-4-carboxy-4-(19-carboxynonadecanoylamino) butyrylamino] ethoxy}ethoxy) acetylamino]ethoxy} ethoxy)acetyl] [DesaminoHis7,Glu22,Arg26, Arg34,Lys37]GLP-1-(7-37); N-epsilon37-(2-{2-[2-((S)-4-carboxy-4-{(S)-4-carboxy-4-[(S)-4-carboxy-4-(19-carboxy-nonadecanoylamino)butyrylamino]butyrylamino} butyrylamino)ethoxy]ethoxy} acetyl)[DesaminoHis7,Glu22,Arg26,Arg34,Lys37]GLP-1-(7-37); N-epsilon37-{2-[2-(2-{(S)-4-[(S)-4-(12-{4-[16-(2-tert-Butyl-2H-tetrazol-5-yl)-hexadecanoylsulfamoyl] butyrylamino}dodecanoylamino)-4-carboxybutyrylamino]-4-carboxybutyrylamino} ethoxy)ethoxy]acetyl}[DesaminoHis7,Glu22,Arg26,Arg34,Lys37] GLP-1 (7-37); N-epsilon37-[2-(2-{2-[2-(2-{2-[(S)-4-carboxy-4-(17-carboxy-heptadecanoylamino)-butyrylamino]-ethoxy}-ethoxy)-acetylamino]-ethoxy}-ethoxy)-acetyl] [Aib8,Glu22, Arg26,Arg34,Lys37]GLP-1-(7-37); N-alpha37-[2-(2-{2-[2-(2-{2-[(S)-4-carboxy-4-(17-carboxy-heptadecanoylamino)-butyrylamino]-ethoxy}-ethoxy)-acetylamino]-ethoxy}-ethoxy)-acetyl] [Aib8,Glu22,Arg26,Arg34,epsilon-Lys37]GLP-1-(7-37)peptide; N-epsilon37-[2-(2-{2-[2-(2-{2-[(S)-4-carboxy-4-(17-carboxy-heptadecanoylamino)-butyrylamino]-ethoxy}-ethoxy)-acetylamino]-ethoxy}-ethoxy)-acetyl] [desaminoHis7, Glu22,Arg26,Arg34,Lys37] GLP-1-(7-37); N-epsilon36-[2-(2-{2-[2-(2-{2-[(S)-4-carboxy-4-(15-carboxy-pentadecanoylamino)-butyrylamino]-ethoxy}-ethoxy)-acetylamino]-ethoxy}-ethoxy)-acetyl] [desaminoHis7, Glu22,Arg26,Glu30,Arg34,Lys36] GLP-1-(7-37)-Glu-Lys peptide; N-epsilon37-[2-(2-{2-[2-(2-{2-[(S)-4-carboxy-4-({trans-4-[(19-carboxynonadecanoylamino)methyl]cyclohexanecarbonyl}amino)butyrylamino]ethoxy}ethoxy)acetylamino]ethoxy}ethoxy)acetyl][Aib8,Glu22,Arg26,Arg34,Lys37]GLP -1-(7-37); N-epsilon37-[2-(2-{2-[2-(2-{2-[(S)-4-carboxy-4-(17-carboxy-heptadecanoylamino)-butyrylamino]-ethoxy}-ethoxy)-acetylamino]-ethoxy}-ethoxy)-acetyl]-[Aib8,Glu22, Arg26,Arg34,Aib35,Lys37]GLP-1-(7-37); N-epsilon37-[(S)-4-carboxy-4-(2-{2-[2-(2-{2-[2-(17-carboxyheptadecanoylamino) ethoxy] ethoxy} acetylamino) ethoxy] ethoxy} acetylamino) butyryl] [Aib8,Glu22,Arg26,34,Lys37] GLP-1 (7-37); N-epsilon37-[2-(2-[2-(2-[2-(2-[4-(17-carboxyheptadecanoylamino)-4(S)-carboxybutyry-lamino]ethoxy)ethoxy]acetylamino)ethoxy]ethoxy)acetyl] [ImPr7,Glu22, Arg26,34,Lys37], GLP-1-(7-37); N-epsilon26-{2-[2-(2-{2-[2-(2-{(S)-4-carboxy-4-[10-(4-carboxyphenoxy) decanoylamino]butyrylamino}ethoxy)ethoxy] acetylamino}ethoxy) ethoxy]acetyl}, N-epsilon37-{2-[2-(2-{2-[2-(2-{(S)-4-carboxy-4-[10-(4-carboxy-phenoxy) decanoylamino] butyrylamino}ethoxy)ethoxy]acetylamino}ethoxy) ethoxy]acetyl}-[Aib8,Arg34,Lys37]GLP-1(7-37)-OH; N-epsilon26 (17-carboxyhepta-decanoyl)-[Aib8,Arg34]GLP-1-(7-37)-peptide; N-epsilon26-(19-carboxynonadecanoyl)-[Aib8,Arg34]GLP-1-(7-37); N-epsilon26-(4-{[N-(2-carboxyethyl)-N-(15-carboxypenta-decanoyl)amino]methyl}benzoyl[Arg34]GLP-1-(7-37); N-epsilon26-[2-(2-[2-(2-[2-(2-[4-(17-carboxyheptadecanoylamino)-4(S)-carboxybutyrylamino]ethoxy)ethoxy] acetylamino) ethoxy]ethoxy)acetyl][Aib8,Arg34]GLP-1-(7-37); N-epsilon26-[2-(2-[2-(2-[2-(2-[4-(19-carboxynonadecanoylamino)-4(S)-carboxybutyrylamino]ethoxy)ethoxy] acetylamino)ethoxy]ethoxy)acetyl][Aib8,Arg34]GLP-1-(7-37); N-epsilon26-[2-(2-[2-(2-[2-(2-[4-(17-carboxyheptadecanoylamino)-4(S)-carboxybutyrylamino]ethoxy)ethoxy] acetylamino)ethoxy]ethoxy)acetyl][3-(4-Imidazolyl)Propionyl7,Arg34]GLP-1-(7-37); N-epsilon26-[2-(2-[2-(2-[2-(2-[4-(17-carboxyheptadecanoylamino)-(carboxymethyl-amino)acetylamino]ethoxy)ethoxy]acetylamino)ethoxy]ethoxy)acetyl][Aib8,Arg34]GLP-1-(7-37); N-epsilon26-[2-(2-[2-(2-[2-(2-[4-(17-carboxyheptadecanoylamino)-3(S)-Sulfopropionylamino]ethoxy)ethoxy]acetylamino)ethoxy]ethoxy)acetyl][Aib8,Arg34]GLP-1-(7-37); N-epsilon26-[2-(2-[2-(2-[2-(2-[4-(17-carboxyheptadecanoylamino)-4(S)-carboxybutyrylamino]ethoxy)ethoxy]acetylamino)ethoxy]ethoxy)acetyl][Gly8,Arg34] GLP-1-(7-37); N-epsilon26-[2-(2-[2-(2-[2-(2-[4-(17-carboxyheptadecanoylamino)-4(S)-carboxybutyrylamino]ethoxy)ethoxy]acetylamino)ethoxy]ethoxy)acetyl][Aib8,Arg34]GLP-1-(7-37)-amide; N-epsilon26-[2-(2-[2-(2-[2-(2-[4-(17-carboxyheptadecanoylamino)-4(S)-carboxybutyrylamino]ethoxy)ethoxy]acetylamino)ethoxy]ethoxy)acetyl] [Aib8,Arg34,Pro37]GLP-1-(7-37)amide; Aib8,Lys26(N-epsilon26-{2-(2-(2-(2-[2-(2-(4-(pentadecanoylamino)-4-carboxybutyrylamino)ethoxy)ethoxy]acetyl)ethoxy) ethoxy)acetyl)}), Arg34)GLP-1 H(7-37)-OH; N-epsilon26-[2-(2-[2-(2-[2-(2-[4-{[N-(2-carboxyethyl)-N-(17-carboxyheptadecanoyl)amino]methyl}benzoyl)amino]ethoxy) ethoxy]acetylamino)ethoxy]ethoxy)acetyl][Aib8,Arg34]GLP-1(7-37); N-alpha7-formyl, N-epsilon26-[2-(2-[2-(2-[2-(2-[4-(17-carboxyheptadecanoyl-amino)-4(S)-carboxy-butyrylamino]ethoxy)ethoxy]acetylamino)ethoxy]ethoxy)acetyl] [Arg34]GLP-1-(7-37); N-epsilon2626-[2-(2-[2-(2-[2-(2-[4-(17-carboxyheptadecanoylamino)-4(S)-carboxy-butyrylamino]ethoxy)ethoxy]acetylamino)ethoxy]ethoxy)acetyl][Aib8, Glu22, Arg34] GLP-1-(7-37); N-epsilon26{3-[2-(2-{2-[2-(2-{2-[2-(2-[4-(15-(N-((S)-1,3-dicarboxypropyl) carbamoyl)pentadecanoylamino)-(S)-4-carboxybutyrylamino] ethoxy)ethoxy] ethoxy}ethoxy)ethoxy]ethoxy}ethoxy)ethoxy]propionyl} [Aib8,Arg34]GLP-1-(7-37); N-epsilon26-[2-(2-[2-(2-[2-(2-[4-{[N-(2-carboxyethyl)-N-(17-carboxy-heptadecanoyl)amino]methyl}benzoyl)amino](4(S)-carboxybutyryl-amino)ethoxy) ethoxy]acetylamino)ethoxy]ethoxy)acetyl][Aib8,Arg34] GLP-1(7-37); N-epsilon26-{(S)-4-carboxy-4-((S)-4-carboxy-4-((S)-4-carboxy-4-((S)-4-carboxy-4-(19-carboxy-nonadecanoylamino)butyrylamino)butyrylamino)butyrylamino) butyrylamino} [Aib8,Arg34]GLP-1-(7-37); N-epsilon26-4-(17-carboxyheptadecanoyl-amino)-4(S)-carboxybutyryl-[Aib8,Arg34]GLP-1-(7-37); N-epsilon26-{3-[2-(2-{2-[2-(2-{2-[2-(2-[4-(17-carboxyheptadecanoylamino)-4(S)-carboxybutyrylamino]ethoxy)ethoxy]ethoxy} ethoxy)ethoxy]ethoxy}ethoxy)ethoxy]propionyl}[Aib8,Arg34]GLP-1-(7-37); N-epsilon26-{2-(2-(2-(2-[2-(2-(4-(17-carboxyheptadecanoylamino)-4-carboxybutyrylamino) ethoxy)ethoxy]acetyl)ethoxy)ethoxy)acetyl)}-[Aib8,22,27,30,35,Arg34,Pro37, Lys26] GLP-1 (7-37)amide; N-epsilon26-[2-(2-[2-[4-(21-carboxyuneicosanoylamino)-4(S)-carboxybutyrylamino]ethoxy]ethoxy)acetyl][Aib8,Arg34]GLP-1-(7-37); and N-epsilon26-[2-(2-[2-(2-[2-(2-[4-(21-carboxyuneicosanoylamino)-4(S)-carboxybutyrylamino] ethoxy)ethoxy]acetylamino)ethoxy]ethoxy)acetyl][Aib8,Arg34]GLP-1-(7-37).

### Delivery agent

A delivery agent or absorption enhancer is for the present invention an excipient capable of increasing the oral exposure of the GLP-1 agonist.

### Salt of N-(8-(2-hydroxybenzoyl)amino)caprylic acid

The delivery agent used in the examples herein is a salt of N-(8-(2-hydroxybenzoyl)amino)caprylic acid (also referred to herein as "NAC"), which contains the anion N-(8-(2-hydroxybenzoyl)amino)caprylate. The structural formula of N-(8-(2-hydroxybenzoyl)amino)caprylate is shown in formula (I).

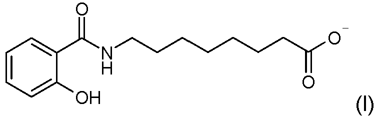

In some embodiments the salt of N-(8-(2-hydroxybenzoyl)amino)caprylic acid comprises one monovalent cation, two monovalent cations or one divalent cation. In an embodiment the salt of N-(8-(2-hydroxybenzoyl)amino)caprylic acid is selected from the group consisting of the sodium salt, potassium salt and/or calcium salt of of N-(8-(2-hydroxybenzoyl)amino)caprylic acid.

In a further embodiment the salt of N-(8-(2-hydroxybenzoyl)amino)caprylic acid is selected from the group consisting of the sodium salt, potassium salt and/or the ammonium salt. In a further embodiment the salt of N-(8-(2-hydroxybenzoyl)amino)caprylic acid is the sodium salt or the potassium salt. Salts of N-(8-(2-hydroxybenzoyl)amino)caprylate may be prepared using the method described in e.g. WO96/030036, WO00/046182, WO01/092206 or WO2008/028859. The salt of N-(8-(2-hydroxybenzoyl)amino)caprylic acid may be crystalline and/or amorphous. In some embodiments the delivery agent comprises the anhydrate, monohydrate, dihydrate, trihydrate, a solvate or one third of a hydrate of the salt of N-(8-(2-hydroxybenzoyl)amino)caprylic acid as well as combinations thereof. In some embodiments the delivery agent is a salt of N-(8-(2-hydroxybenzoyl)amino)caprylic acid as described in WO2007/121318.

In a further embodiment the delivery agent is sodium N-(8-(2-hydroxybenzoyl)amino)caprylate (referred to as "SNAC" herein) and also known as sodium 8-(salicyloylamino)octanoate.

### Composition

The composition or pharmaceutical composition of the present invention is a solid or dry composition suited for administration by the oral route as described further herein below.

In some embodiments the composition comprises at least one pharmaceutically acceptable excipient. The term **"excipient"** as used herein broadly refers to any component other than the active therapeutic ingredient(s) or active pharmaceutical ingredient(s) (API(s)). The excipient(s) may be a pharmaceutically inert substance, an inactive substance, and/or a therapeutically or medicinally none active substance.

The excipient(s) may serve various purposes, e.g. as a carrier, vehicle, filler, binder, lubricant, glidant, disintegrant, flow control agent, crystallization inhibitors solubilizer, stabilizer, colouring agent, flavouring agent, surfactant, emulsifier or combinations of thereof and/or to improve administration and/or to improve absorption of the therapeutically active substance(s) or active pharmaceutical ingredient(s). The amount of each excipient used may vary within ranges conventional in the art. Techniques and excipients which may be used to formulate oral dosage forms are described in Handbook of Pharmaceutical Excipients, 8th edition, Sheskey et al., Eds., American Pharmaceuticals Association and the Pharmaceutical Press, publications department of the Royal Pharmaceutical Society of Great Britain (2017); and Remington: the Science and Practice of Pharmacy, 22nd edition, Remington and Allen, Eds., Pharmaceutical Press (2013).

In some embodiments the excipients may be selected from **binders,** such as polyvinyl pyrrolidone (povidone), etc.; **fillers** such as cellulose powder, microcrystalline cellulose (MCC), cellulose derivatives like hydroxymethylcellulose, hydroxyethylcellulose, hydroxypropylcellulose and hydroxy-propylmethylcellulose, dibasic calcium phosphate, corn starch, pregelatinized starch, etc.; **disintegrants** such as cellulose powder, microcrystalline cellulose, sodium starch glycolate, polacrilin potassium, crospovidon, croscarmellose, sodium carboxymethylcellulose, dried corn starch, etc.; **lubricants** and/or **glidants** such as stearic acid, magnesium stearate, sodium stearylfumarate, glycerol tribehenate, etc.; **flow control agents** such as colloidal silica, talc, etc.; **crystallization inhibitors** such as povidone, etc.; **solubilizers** such as pluronic, povidone, etc.; **colouring agents,** including dyes and pigments such as iron oxide red or yellow, titanium dioxide, talc, etc.; **pH control agents** such as citric acid, tartaric acid, fumaric acid, sodium citrate, dibasic calcium phosphate, dibasic sodium phosphate, etc.; **surfactants** and **emulsifiers** such as pluronic, polyethylene glycols, sodium carboxymethyl cellulose, polyethoxylated and hydrogenated castor oil, etc.; and mixtures of two or more of these excipients and/or adjuvants; **hydrotropes** such as Nipecotamide, Nicotinamide, p-hydroxybenzoic acid sodium, N,N dimethyl urea, N,N dimethyl benzamide, N,N diethyl nicotinamide, Sodium salicylate, Resorcinol, Sodium benzoate, Sodium Xylenesulfonate, Sodium p-toluenesulfonate, 1-Methylnicotinamide, Pyrogallol, Pyrocathecol, Epigallocatechin gallate, Tannic acid and Gentisic acid sodium salt hydrate.

The composition may comprise a **binder,** such as povidone; starches; celluloses and derivatives thereof, such as microcrystalline cellulose, e.g., Avicel PH, hydroxypropyl cellulose hydroxylethyl cellulose and hydroxylpropylmethyl cellulose, sucrose, dextrose, corn syrup, polysaccharides, and gelatin. The **binder** may be selected from the group consisting of dry binders and/or wet granulation binders. Suitable dry binders are, e.g., cellulose powder and microcrystalline cellulose, such as Avicel. In some embodiments the composition comprises Avicel, such as Aavicel PH 101. Suitable binders for wet granulation or dry granulation are corn starch, polyvinyl pyrrolidone (povidon), vinylpyrrolidone-vinylacetate copolymer (copovidone) and cellulose derivatives like hydroxymethylcellulose, hydroxyethylcellulose, hydroxypropylcellulose and hydroxyl-propylmethylcellulose. In some embodiments the composition comprises povidone.

In some embodiments the composition comprises a **filler** which may be selected from lactose, mannitol, erythritol, sucrose, sorbitol, calcium phosphate, such as calciumhydrogen phosphate, microcrystalline cellulose (MCC), powdered cellulose, confectioner's sugar, compressible sugar, dextrates, dextrin and dextrose. In some embodiments the composition comprises microcrystalline cellulose, such as Avicel PH 101 or Avicel PH 200.

In some embodiments the composition comprises a lubricant and/or a glidant. In some embodiments the composition comprises a **lubricant** and/or a **glidant,** such as talc, magnesium stearate, calcium stearate, zinc stearate, glyceryl behenate, glyceryl debehenate, behenoyl polyoxyl-8 glycerides, polyethylene oxide polymers, sodium lauryl sulfate, magnesium lauryl sulfate, sodium oleate, sodium stearyl fumarate, stearic acid, hydrogenated vegetable oils, silicon dioxide and/or polyethylene glycol etc. In some embodiments the composition comprises magnesium stearate.

In some embodiments the composition comprises a **disintegrant,** such as sodium starch glycolate, polacrilin potassium, sodium starch glycolate, crospovidon, croscarmellose, sodium carboxymethylcellulose or dried corn starch, or a **hydrotrope,** such as nicotinamide or Resorcinol. The composition may comprise one or more **surfactants,** for example a surfactant, at least one surfactant, or two different surfactants. The term "surfactant" refers to any molecules or ions that are comprised of a water-soluble (hydrophilic) part, and a fat-soluble (lipophilic) part. The surfactant may e.g. be selected from the group consisting of anionic surfactants, cationic surfactants, nonionic surfactants, and/or zwitterionic surfactants.

In a broad aspect the invention relates to a solid pharmaceutical composition comprising a GLP-1 agonist and a stability enhancer. As demonstrated herein histidine has been found to work as a stability enhancer as it is an efficient aldehyde scavenger in compositions with a GLP-1 agonist susceptible for aldehyde mediated degradation leading to accumulation of impurities overtime. Alternative aldehyde scavengers may be selected from a larger group of chemical compounds suitable as excipients in pharmaceutical compositions.

The description here below also refers to compositions comprising or consisting of specific ingredients, i.e. a GLP-1 agonist, a delivery agent and histidine and optionally one or more excipients, such as a filler, a binder, a disintegrant or hydrotrope, and/or a lubricant. The term consisting is to be understood to nevertheless encompass trace amounts of any substance with no effect on the function of the composition. Such substances can be impurities remaining in preparation of the GLP-1 agonist, from the production of the salt of NAC, the histidine preparation or minimal amounts (below 1 %) of any pharmaceutical acceptable excipient that do not affect the quality of the formulation.

In one embodiment the pharmaceutical composition comprises a balanced amount of histidine relative to the amount of the GLP-1 agonist. The effect of histidine has been observed over a range of concentrations.

In one embodiment the moles of histidine are at least equal to half the moles of the GLP-1 agonist, in other words, the molar ratio of histidine to the GLP-1 agonist is at least 0.5. In one embodiment the molar ratio of histidine to GLP-1 agonist is at least 0.6, or such as at least 0.7, or such as at least 0.8, or such as at least 0.9, or such as at least 1.

In one embodiment the moles of histidine are at most 100 times more than the moles of the GLP-1 agonist, in other words, the molar ratio of histidine to GLP-1 agonist is at most 100. In one embodiment the molar ratio of histidine to GLP-1 agonist is at most 80 or such as at most 60, or such as at most 40 or 30.

In one embodiment the molar ratio of histidine to the GLP-1 agonist is from 0.5-100, such as 0.6-80, such as 0,7-60, such as 0,75-50, such as 0.8-40, or such as 0.9-30.

In embodiments where the GLP-1 agonist has a molar mass around 4000 g/mol the mass ratio of histidine to the GLP-1 agonist is at least 0.02, such as a least 0.03 or 0.04. In a further such embodiment the mass ratio of histidine to the GLP-1 agonist is at most 4, such as at most 3, such as at most 2 or 1.

In one embodiment the mass ratio of histidine to a GLP-1 agonist with a Mw around 4000 g/mol is from 0.02-4, such as 0,02-3, such as 0.02-2, such as 0.03-1, such as 0.04-1, or such as 0.05-1.

The pharmaceutical composition according to the invention is preferably in a dosage form suitable for oral administration as described herein below. In the following the absolute amounts of the ingredients of the composition of the invention are provided with reference to the content in a dose unit i.e. per tablet, capsule or sachet.

The pharmaceutical compositions of the invention may in a further embodiment comprise at most 1000 mg of said salt of N-(8-(2-hydroxybenzoyl)amino)caprylic acid (NAC) per dose unit. In one embodiment the invention relates to a composition wherein a dose unit comprises at most 600 mg of said salt.

In some embodiments the amount of the salt of N-(8-(2-hydroxybenzoyl) amino)caprylic acid per dose unit is at least 0.15 mmol, such as at least 0.20 mmol, at least 0.25 mmol, at least 0.30 mmol, at least 0.35 mmol, at least 0.40 mmol, at least 0.45 mmol, at least 0.50 mmol, at least 0.55 mmol, at least 0.60 mmol, at least 0.65 mmol, at least 0.75 mmol or at least 0.8 mmol.

In some embodiments the amount of the salt of N-(8-(2-hydroxybenzoyl)amino)caprylic acid per dose unit of the composition is up to 2.5 mmol, such as up to 2.0 mmol, such as up to 1.5 mmol, up to 1 mmol, up to 0.75 mmol, up to 0.6 mmol, up to 0.5 mmol, up to 0.4 mmol, up to 0.3 mmol or up to 0.2 mmol.

In some embodiments the amount of the salt of N-(8-(2-hydroxybenzoyl) amino)caprylic acid per dose unit of the composition is in the range of 0.20-2.5 mmol, 0.25-1.5 mmol.

In some embodiments the amount of the salt of N-(8-(2-hydroxybenzoyl) amino)caprylic acid per dose unit of the composition is in the range of 0.2-0,6 mmol, 0.8-1.4 mmol or 1.4-2.0.

In some embodiment the delivery agent is sodium N-(8-(2-hydroxybenzoyl)amino)caprylate (SNAC").

In some embodiments the amount of SNAC per dose unit of the composition is at least 50 mg, such as at least 75 mg, at least 100 mg, at least 125 mg, at least 150 mg, at least 175 mg, at least 200 mg, at least 225 mg, at least 250 mg, at least 275 m , at least 300 mg, at least 350 mg, at least 450 mg or at least 500 mg.

In some embodiments the amount of SNAC per dose unit of the composition is up to 750 mg, such as up to 700 mg, such as up to 650 mg, such as up to 600 mg, such as up to 550 mg, up to 525 mg, up to 500 mg, up to 475 mg, up to 450 mg, up to 425 mg, up to 400 mg, up to 375 mg, up to 350 mg, up to 300 mg, up to 250 mg, up to 200 mg, or up to 150 mg per dose unit, .

In some embodiments the amount of SNAC per dose unit of the composition is in the range of 50-750 mg, 65-600 mg, such as from 80-550 mg

In some embodiments the amount of SNAC per dose unit of the composition is in the range of 50-150 mg, 150-500 mg, such as from 200-400 mg, such as 300-700, or such as 400 to 600 mg.

In an embodiment, a dose unit of the pharmaceutical compositions of the invention comprises 0.5-100 mg of the GLP-1 agonist, such as 0.5-75 mg, such as 0.5-60 mg.

In some embodiments a dose unit of the composition comprises an amount of GLP-1 agonist is in the range of 0.5 - 50 mg, 1 to 50 mg, 2 to 50 mg or 4to 40 mg.

In some embodiments a dose unit of the composition comprises an amount of GLP-1 agonist is in the range of 0.5 - 50 mg, 0.5 - 40 mg, 0.5 - 30 mg or 0.5 - 20 mg.

In some embodiments a dose unit comprises 0.5-10 mg of the GLP-1 agonist, such as 0.75- 4 ½ mg, such as 1, 1 ½, 2, 2 ½ or 3 mg or 3 ½, 4, 4 ½ mg, such as 1-3 or 3-5 mg of the GLP-1 agonist per dose unit.

In some embodiments a dose unit comprises 2 to 20 mg of the GLP-1 agonist, such as 2-15 mg, such as 2, 3, 4 or 5 mg, or such as 8, 10, 12 or 14 mg, such as 15 mg or such as 20 mg of the GLP-1 agonist.

In some embodiments a dose unit comprises 5 to 75 mg of the GLP-1 agonist, such as 10-60 mg, such as 20, 30, 40 or 50 mg, or such as 25, 35, or 45 mg, or such as 30-50 mg or such as 20-40 mg of the GLP-1 agonist.

As described above the amount of the histidine is to be balanced with the amount of the GLP-1 agonist but in general a dose unit of the compositions of the invention comprises 0.01-400 mg of histidine. In an embodiment a dose unit comprises 0.01-300 mg, such as 0.02-300 mg, such as 0.05-200 mg , such as 0.02-100 mg or such as 1-50 mg histidine.

In one embodiment a dose unit of the composition according to the invention comprises:
i) 0.5-100 mg GLP-1 agonist, such as Semaglutide, GLP-1 agonist B or GLP-1 agonist C,
ii) 50-750 mg salt of N-(8-(2-hydroxybenzoyl)amino)caprylic acid (NAC), such as the sodium salt of NAC (SNAC) and
iii) 0.01-200 mg histidine.

In one embodiment a dose unit of the composition according to the invention comprises:
i) 1-100 mg Semaglutide
ii) 50-150 mg salt of N-(8-(2-hydroxybenzoyl)amino)caprylic acid (NAC), such as the sodium salt of NAC (SNAC) and
iii) 0.02-100 mg, such as 1-50 mg histidine

In one embodiment a dose unit of the composition according to the invention comprises:
i) 1-100 mg Semaglutide
ii) 150-500 mg salt of N-(8-(2-hydroxybenzoyl)amino)caprylic acid (NAC), such as the sodium salt of NAC (SNAC) and
iii) 0.02-100 mg , such as 1-50 mg histidine

In one embodiment a dose unit of the composition according to the invention comprises:
i) 1-100 mg Semaglutide
ii) 350-600 mg salt of N-(8-(2-hydroxybenzoyl)amino)caprylic acid (NAC), such as the sodium salt of NAC (SNAC) and
iii) 0.02-100 mg, such as 1-50 mg histidine

As described herein above the compositions may further comprise pharmaceutical excipient(s), such as a lubricant, a binder, a disintegrant or hydrotrope and/or a filler.

In one embodiment a dose unit of the composition according to the invention comprises:
i) 0.5-100 mg GLP-1 agonist, such as Semaglutide, GLP-1 agonist B or GLP-1 agonist C,
ii) 50-750 mg salt of N-(8-(2-hydroxybenzoyl)amino)caprylic acid (NAC), such as the sodium salt of NAC (SNAC),
iii) 0.01-200 mg histidine and
iv) optionally further pharmaceutical excipient(s).

In one embodiment a dose unit of the composition according to the invention comprises:
i) 0.5-100 mg GLP-1 agonist, such as Semaglutide, GLP-1 agonist B or GLP-1 agonist C,
ii) 50-750 mg salt of N-(8-(2-hydroxybenzoyl)amino)caprylic acid (NAC), such as the sodium salt of NAC (SNAC),
iii) 0.01-200 mg histidine
iv) a lubricant and
v) optionally further pharmaceutical excipient(s).

In one embodiment a dose unit of the composition according to the invention comprises:
i) 0.5-100 mg GLP-1 agonist, such as Semaglutide, GLP-1 agonist B or GLP-1 agonist C.
ii) 50-750 mg salt of N-(8-(2-hydroxybenzoyl)amino)caprylic acid (NAC), such as the sodium salt of NAC (SNAC),
iii) 0.01-200 mg histidine,
iv) a lubricant,
v) a disintegrant or hydrotrope and
vi) optionally further pharmaceutical excipient(s).

In one embodiment a dose unit of the composition according to the invention comprises:
i) 0.5-100 mg GLP-1 agonist, such as Semaglutide, GLP-1 agonist B or GLP-1 agonist C.
ii) 50-750 mg salt of N-(8-(2-hydroxybenzoyl)amino)caprylic acid (NAC), such as the sodium salt of NAC (SNAC),
iii) 0.01-200 mg histidine,
iv) a lubricant,
v) a disintegrant or hydrotrope,
vi) a filler and
vii) optionally further pharmaceutical excipient(s).

In one embodiment a dose unit of the composition according to the invention comprises:
i) 0.5-100 mg GLP-1 agonist, such as Semaglutide, GLP-1 agonist B or GLP-1 agonist C.
ii) 50-750 mg salt of N-(8-(2-hydroxybenzoyl)amino)caprylic acid (NAC), such as the sodium salt of NAC (SNAC),
iii) 0.01-200 mg histidine,
iv) a lubricant,
v) a disintegrant or hydrotrope,
vi) a filler,
vii) a binder and
viii) optionally further pharmaceutical excipient(s).

In one embodiment a dose unit of the composition according to the invention comprises:
i) 0.5-100 mg GLP-1 agonist, such as Semaglutide, GLP-1 agonist B or GLP-1 agonist C,
ii) 50-750 mg salt of N-(8-(2-hydroxybenzoyl)amino)caprylic acid (NAC), such as the sodium salt of NAC (SNAC),
iii) 0.01-200 mg histidine,
iv) a lubricant, such as magnesium stearate,
v) a filler, such as MCC,
vi) a disintegrant or hydrotrope, such as nicotinamide,
vii) a binder, such as povidone, and
viii) optionally further pharmaceutical excipient(s).

In further embodiment the amounts of the various pharmaceutical excipients maybe adjusted per dose unit, or relative to one or more of the other ingredients.

In one embodiment the composition comprises less than 5 % w/w lubricant of the amount of total amount of excipients.

In one embodiment the composition comprises less than 5 % w/w lubricant of the amount of the delivery agent. In one embodiment the composition comprises 0.25-5 % w/w, such as 1-4 % w/w lubricant of the amount of the delivery agent. In further embodiments the composition comprises 0.25- 5 % w/w, such as 1-4 % w/w lubricant of the amount of salt of NAC, such as SNAC.

In one embodiment a dose unit of the composition comprises 0.1-20 mg lubricant or 1-15 mg lubricant, such as magnesium stearate

In one embodiment a dose unit of the composition according to the invention comprises 20-150 mg filler or 40-100 mg filler such as MCC.

In one embodiment a dose unit of the composition comprises 10-600 mg, such as 10-500 mg disintegrant or hydrotrope. In one embodiment a dose unit of the composition comprises 10-600 mg, such as 10-500 mg, such as 10-400 mg such as 20-300 mg or such as 25-250 mg nicotinamide.

In one embodiment a dose unit of the composition according to the invention comprises 1-30 mg binder, 1-25 mg binder or 1-20 mg binder, such as povidone.

### Dosage form

The composition may be administered in several dosage forms, for example as a tablet; a coated tablet; a sachet or a capsule such as hard or soft capsules and all such compositions are considered solid oral dosage forms.

The composition may be in the form of a dose unit, such as a tablet. In some embodiments the weight of the dose unit is in the range of 50 mg to 1000 mg, such as in the range of 50-750 mg, or such as in the range of 100-600 mg.

In some embodiments the weight of the dose unit is in the range of 50 mg to 400 mg, such as in the range of 75-350 mg or 100-300 mg.

In some embodiments the weight of the dose unit is in the range of 200 mg to 600 mg, such as in the range of 250-550 mg or 300-500 mg.

In some embodiments the weight of the dose unit is in the range of 300 mg to 700 mg, such as in the range of 350-650 mg or 400-600 mg.

In some embodiments the weight of the dose unit is in the range of 400 mg to 1000 mg, such as in the range of 450-850 mg or 500-700 mg.

In some embodiments the composition, or some of the ingredients of the composition, may be granulated prior to being compressed to tablets or filled into capsules or sachets.

The composition may comprise a granular part and/or an extra-granular part, wherein the granular part has been granulated and the extra-granular part has been added after granulation.

The granular part may comprise one or more types of granules comprising different subset of the ingredients of the composition.

The granular part(s) may comprise the GLP-1 agonist, the delivery agent and/or histidine. In an embodiment the granular part(s) may comprise one or more further excipient, such as a lubricant, a filler, a disintegrant, a hydrotrope, and/or a binder.

In an embodiment a granular part comprises the delivery agent and the lubricant. In an embodiment a granular part comprises SNAC and magnesium stearate.

In an embodiment histidine is included in the granular part or the extra-granular part.

In an embodiment the GLP-1 agonist is included in the extra-granular part. In one embodiment the extra-granular part comprises the GLP-1 agonist. In an embodiment the extra-granular part may further comprise histidine. In one embodiment the extra-granular part further comprises a lubricant, such as magnesium stearate.

### Preparation of composition

Preparation of a composition according to the invention may be performed according to methods known in the art.

To prepare a dry **blend** of materials, the various components are weighed, optionally delumped or sieved and then combined. The mixing of the components may be carried out until a homogeneous blend is obtained.

The term "granules" refers broadly to pharmaceutical ingredients in the form of particles, granules and aggregates which are used in the preparation of solid dose formulations. Generally, granules are obtained by processing a powder or a blend to obtain new combined particles of the desired size.

If granules are to be used in the further processing into tablets, capsules, or sachets, granules may be produced in a manner known to a person skilled in the art, for example using **wet granulation** methods known for the production of "built-up" granules or "broken-down" granules. Methods for the formation of **built-up granules** may operate continuously and comprise, for example simultaneously spraying the granulation mass with granulation solution and drying, for example in a drum granulator, in pan granulators, on disc granulators, in a fluidized bed, by spray-drying or spray-solidifying, or operate discontinuously, for example in a fluidized bed, in a rotary fluid bed, in a batch mixer, such as a high shear mixer or a low shear mixer, or in a spray-drying drum such as an apparatus from the companies Loedige, Glatt, Diosna, Fielder, and Collette. Methods for the production of **broken-down granules,** which may be carried out discontinuously and in which the granulation mass first forms a wet aggregate with the granulation solution, which is subsequently comminuted or by other means formed into granules of the desired size and the granules may then be dried. Suitable equipment for the granulation step are planetary mixers, low shear mixers, high shear mixers, extruders and spheronizers, such as an apparatus from the companies Loedige, Glatt, Diosna, Fielder, Collette, Aeschbach, Alexanderwerk, Ytron, Wyss & Probst, Werner & Pfleiderer, HKD, Loser, Fuji, Nica, Caleva and Gabler. Granules may be also formed by **dry granulation** techniques in which one or more of the excipient(s) and/or the active pharmaceutical ingredient is compressed to form relatively large moldings, for example slugs or ribbons, which are comminuted by grinding into the granules of the desired size, and the ground material serves as the material for further processing into tablets, capsules, or sachets. Suitable equipment for dry granulation is an apparatus from the companies Gerteis, Alexanderwerk, and Freund-Vector.

Further methods of obtaining granules can include hot melt extrusion, spray drying, spray granulation and/or ball milling.

In an embodiment the invention relates to a composition comprising
i. a GLP-1 agonist,
ii. a salt of N-(8-(2-hydroxybenzoyl)amino)caprylic acid (NAC),
iii. histidine and
iv. a lubricant, such as magnesium stearate.
wherein the composition comprises a granulate of ii) and iv).

In embodiments where the granular part comprises delivery agent and lubricant these excipients may be co-processed prior to or in the preparation of the granules.

The granulation may be obtained by various methods as described above, wherein ii) and iv) are initially mixed either as powders or by the preparation of a solution comprising both ingredients.

Granules of ii) and iv) may be obtained by wet granulation or dry granulation of the solution or blend.

In one embodiment i) and iii) are co-processed prior to tablet compression.

In one embodiment a solution or suspension of i) and iii) is prepared and subjected to spray drying or spray granulation whereby a powder or granules hereof are directly obtained. In one embodiment spray drying can be used followed by dry granulation/roller compaction to obtain the granules. Alternatively, i) and iii) can be prepared as a mixture.

In order to obtain a homogenous product one or more sieving step(s) can be included prior to the final dry granulation step/roller compaction or tablet compression.

To **compact** the material into a solid oral dosage form, for example a tablet, a tablet press may be used. In a tablet press, the tabletting material is filled (e.g. force fed or gravity fed) into a die cavity. The tabletting material is then compacted by a set of punches applying pressure. Subsequently, the resulting tablet is ejected from the tablet press. The above mentioned tabletting process is subsequently referred to herein as the "compression process". Suitable **tablet presses** include, rotary tablet presses and eccentric tablet presses. Suitable equipment for tablet compression is an apparatus from the companies Fette, Korsch, GEA Courtouy, and Manesty.

In some embodiments the invention relates to a method of preparation of a composition, such as a tablet according to the invention. In one embodiment the method of preparing a tablet comprises;
a) granulating a delivery agent and optionally a lubricant
b) blending of the granulate of a) with a GLP-1 agonist and histidine and
c) compression of the blend into tablets.

The granulation may be a wet or dry granulation. As described above the lubricant may be magnesium stearate which may in addition to step a, also be include in one or more of steps b) and c).

In one embodiment the invention relates to a method for producing a solid pharmaceutical composition comprising the steps of;
i) obtaining a blend comprising a GLP-1 agonist and histidine,
ii) co-processing the blend of i) and
iii) preparing said solid pharmaceutical composition using the product of ii).

In one embodiment the method is for producing a solid pharmaceutical composition comprising the steps of;
i) obtaining a solution or suspension comprising a GLP-1 agonist and histidine,
ii) spray-drying of i) and
iii) preparing said solid pharmaceutical composition using the product of ii).

In all of the above embodiments, the method may further include preparing said solid pharmaceutical composition using one or more pharmaceutical excipient(s), such as a lubricant, a binder, a disintegrant or hydrope, and/or a filler as described above.

### Pharmaceutical Indications

The present invention also relates to a composition of the invention for use as a medicament. In particular embodiments the composition of the invention may be used for the following medical treatments, all preferably relating one way or the other to diabetes:
(i) prevention and/or treatment of all forms of diabetes, such as hyperglycemia, type 2 diabetes, impaired glucose tolerance, type 1 diabetes, non-insulin dependent diabetes, MODY (maturity onset diabetes of the young), gestational diabetes, and/or for reduction of HbA1C;
(ii) delaying or preventing diabetic disease progression, such as progression in type 2 diabetes, delaying the progression of impaired glucose tolerance (IGT) to insulin requiring type 2 diabetes, and/or delaying the progression of non-insulin requiring type 2 diabetes to insulin requiring type 2 diabetes;
(iii) improving β-cell function, such as decreasing β-cell apoptosis, increasing β-cell function and/or β-cell mass, and/or for restoring glucose sensitivity to β-cells;
(iv) prevention and/or treatment of cognitive disorders;
(v) prevention and/or treatment of eating disorders, such as obesity, e.g. by decreasing food intake, reducing body weight, suppressing appetite, inducing satiety; treating or preventing binge eating disorder, bulimia nervosa, and/or obesity induced by administration of an antipsychotic or a steroid; reduction of gastric motility; and/or delaying gastric emptying;
(vi) prevention and/or treatment of diabetic complications, such as neuropathy, including peripheral neuropathy; nephropathy; or retinopathy;
(vii) improving lipid parameters, such as prevention and/or treatment of dyslipidemia, lowering total serum lipids; lowering HDL; lowering small, dense LDL; lowering VLDL: lowering triglycerides; lowering cholesterol; increasing HDL; lowering plasma levels of lipoprotein a (Lp(a)) in a human; inhibiting generation of apolipoprotein a (apo(a)) in vitro and/or in vivo;
(iix) prevention and/or treatment of cardiovascular diseases, such as syndrome X; atherosclerosis; myocardial infarction; coronary heart disease; stroke, cerebral ischemia; an early cardiac or early cardiovascular disease, such as left ventricular hypertrophy; coronary artery disease; essential hypertension; acute hypertensive emergency; cardiomyopathy; heart insufficiency; exercise tolerance; chronic heart failure; arrhythmia; cardiac dysrhythmia; syncopy; atheroschlerosis; mild chronic heart failure; angina pectoris; cardiac bypass reocclusion; intermittent claudication (atheroschlerosis oblitterens); diastolic dysfunction; and/or systolic dysfunction;
(ix) prevention and/or treatment of gastrointestinal diseases, such as inflammatory bowel syndrome; small bowel syndrome, or Crohn's disease; dyspepsia; and/or gastric ulcers;
(x) prevention and/or treatment of critical illness, such as treatment of a critically ill patient, a critical illness poly-nephropathy (CIPNP) patient, and/or a potential CIPNP patient; prevention of critical illness or development of CIPNP; prevention, treatment and/or cure of systemic inflammatory response syndrome (SIRS) in a patient; and/or for the prevention or reduction of the likelihood of a patient suffering from bacteraemia, septicaemia, and/or septic shock during hospitalisation; and/or
(xi) prevention and/or treatment of polycystic ovary syndrome (PCOS).

In an embodiment, the indication is selected from the group consisting of (i)-(iii) and (v)-(iix), such as indications (i), (ii), and/or (iii); or indication (v), indication (vi), indication (vii), and/or indication (iix). In another embodiment, the indication is (i). In a further embodiment the indication is (v). In a still further embodiment, the indication is (iix). In some embodiments the indications are type 2 diabetes and/or obesity.

The invention further relates to a method of treatment of an individual in need thereof, comprising administering a therapeutically active amount of a composition according to the present invention to said individual. In a further such embodiments one or more dose units may be administered to said individual in need.

### Method of treatment

The invention further relates to a method of treating a subject in need thereof, comprising administering a therapeutically effective amount of a composition according to the present invention to said subject. In one embodiment the method of treatment is for treatment of diabetes or obesity and/or the further indications specified above.

In some embodiments, a method for treating diabetes is described comprising administering to a subject in need thereof a therapeutically effective amount of a pharmaceutical composition comprising a GLP-1 agonist, a salt of N-(8-(2-hydroxybenzoyl)amino)caprylic acid (NAC), histidine and optionally excipient such as a filler, a binder, a disintegrant, and/or a lubricant.

In some embodiments, a method for treating diabetes is described comprising administering to a subject in need thereof a therapeutically effective amount of a pharmaceutical composition comprising;
i) 0.5-100 mg mg GLP-1 agonist, such as Semaglutide, GLP-1 agonist B or GLP-1 agonist C.
ii) 50-750 mg salt of N-(8-(2-hydroxybenzoyl)amino)caprylic acid (NAC), such as the sodium salt of NAC (SNAC) and
iii) 0.01-200 mg histidine and
iv) 0-10 mg lubricant.

In some embodiments, the GLP-1 agonist is semaglutide having a formula of N-epsilon26-[2-(2-{2-[2-(2-{2-[(S)-4-carboxy-4-(17-carboxy-heptadecanoylamino)butyrylamino]ethoxy}ethoxy)acetylamino]ethoxy}ethoxy)acetyl] [Aib8,Arg34]GLP-1(7-37).

In some embodiments, the GLP-1 agonist is *N^{ε26}*{2-[2-(2-{2-[2-(2-{(S)-4-Carboxy-4-[10-(4-carboxyphenoxy)decanoylamino]butyrylamino}-ethoxy)ethoxy]acetylamino}ethoxy)ethoxy]acetyl}, *N^{ε37}*-{2-[2-(2-{2-[2-(2-{(S)-4-carboxy-4-[10-(4-carboxyphenoxy)decanoylamino]butyrylamino}ethoxy)ethoxy]acetylamino}ethoxy)ethoxy]-acetyl}-[Aib⁸,Arg³⁴,Lys³⁷]GLP-1(7-37) (GLP-1 agonist B).

In some embodiments, the GLP-1 agonist is N^{ε27}-[2-[2-[2-[[2-[2-[2-[[(4S)-4-carboxy-4-[10-(4-carboxyphenoxy)decanoylamino]butanoyl]amino] ethoxy]ethoxy]acetyl]amino]ethoxy]ethoxy]-acetyl], N^{ε36}-[2-[2-[2-[[2-[2-[2-[[(4S)-4-carboxy-4-[10-(4-carboxyphenoxy)decanoylamino]-butanoyl]amino]ethoxy]ethoxy]acetyl]amino]ethoxy]ethoxy]acetyl]-[Aib8,Glu22,Arg26,Lys27, Glu30,Arg34,Lys36]-GLP-1-(7-37)-peptidyl-Glu-Gly (GLP-1 agonist C).

In one embodiment the composition is administered orally and is in a form of a table, capsule or a sachet.

In a further such embodiments one or more dose units may be administered to said subject in need.

### Combination treatment

The treatment with a composition according to the present invention may also be combined with one or more additional active pharmaceutical ingredient(s), e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: Insulin, sulphonylureas, biguanides, meglitinides, glucosidase inhibitors, glucagon antagonists, DPP-IV (dipeptidyl peptidase-IV) inhibitors, sodium glucose linked transporter 2 (SGLT2) inhibitors; canagliflozin, dapagliflozin, empagliflozin, ertugliflozin, ipragliflozin, tofogliflozin, luseogliflozin, bexagliflozin, remogliflozin etabonate and sotagliflozin, particulally dapagliflozin and empagliflozin, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), Gastric Inhibitory Polypeptides (GIP analogues), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, PYY agonists, Y2 receptor agonists, Y4 receptor agonists, mixed Y2/Y4 receptor agonists, MC4 (melanocortin 4) agonists, orexin antagonists, TNF (tumour necrosis factor) agonists, CRF (corticotropin releasing factor) agonists, CRF BP (corticotropin releasing factor binding protein) antagonists, urocortin agonists, β3 agonists, oxyntomodulin and analogues, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists, Gastric Inhibitory Polypeptide agonists or antagonists (GIP analogues), gastrin and gastrin analogues.

### EXAMPLES

### Materials and Methods

### Method 1 - Co-spray-drying of semaglutide and histidine

Water solutions or ethanol-water solutions (1:9) were prepared by dissolving semaglutide and histidine in molar ratios around 1:1, 1:2, 1:3.75, 1:7.5, 1:15, 1:30, 1:32, and 1:100, respectively, resulting in concentrations around 2-2.5 % w/w. The solutions were spray-dried (Büchi Mini Spray Drier) with the aspirator set at 100 %, an inlet temperature around 120 or 155 °C, an outlet temperature around 45 or 78 °C, and a solution feed flow around 6 or 12 g/min.

### Method 2 - Dry granulation of SNAC and magnesium stearate

SNAC and magnesium stearate were mixed at 25 rpm for 50 min using a Pharmatech MB blender prior to dry granulation. Dry granulation was carried out by roller compaction on a Gerteis MINI-PACTOR using smooth rolls, a 0.63 or 0.80 mm screen, and a granulator speed of 30-120 rpm. Roll speed was set at 3-6 rpm and roller compaction forces at 6-9 kN/cm were applied at a gap of 1-2 mm. Subsequent to dry granulation comminution of the moldings into granules was carried out.

### Assay I: Analysis of semaglutide related impurities - RP-UHPLC method

Samples were prepared by dissolving tablets using a phosphate buffer pH 7.4 (50 mM KH₂PO₄, 380 mM potassium chloride) mixed with 20 % (v/v) acetonitrile and optionally with 0.1 % (w/v) Brij^{®}35 (polyoxyethylene lauryl ether) as extraction buffer. Sample content of semaglutide related impurities was determined using a RP-UHPLC method. The UHPLC method was based on gradient elution on a C18 column. The eluent system was 0.09 M (NH4)2HPO4 (pH 3.6), acetonitrile and 2-propanol in water with UV detection at 215 nm.

Hydrophobic impurities group 1 were calculated as % based on the area of all semaglutide related peaks eluted after the end of the semaglutide peak and to the start of the 2nd linear gradient relative to the total area of all semaglutide related peaks in in the chromatogram.

Hydrophobic impurities group 2 were calculated as % based on the area of all semaglutide related peaks eluted in the 2nd linear gradient relative to the total area of all semaglutide related peaks in in the chromatogram.

### Assay II: Analysis of semaglutide aldehyde adducts - LC-MS method

Samples were prepared by dissolving mixtures or tablets using a phosphate buffer pH 7.4 (50 mM KH2PO4) mixed with 20 % (v/v) acetonitrile. Sample content of semaglutide aldehyde adducts was determined using a high-resolution mass spectrometer equipped with RP-UHPLC. The UHPLC method was based on gradient elution on a C18 column. The eluent system was 0.06 % TFA, 90 % acetonitrile in water with UV detection at 214 nm.

From the total ion chromatograms (TIC), reconstructed ion chromatograms (RIC) were extracted of the triple charged monoisotopic peaks of semaglutide and the +12 and +26 impurities, i.e. semaglutide formaldehyde adduct and semaglutide acetaldehyde adduct, respectively. The RICs peaks were integrated and the area was used to calculate the levels of +12 and +26 relative to semaglutide.

### Assay III: Analysis of semaglutide aldehyde adducts - RP-UHPLC method

Samples were prepared by dissolving tablets using a phosphate buffer pH 7.4 (50 mM KH₂PO₄, 380 mM potassium chloride) mixed with 20 % (v/v) acetonitrile and optionally with 0.1 % (w/v) Brij^{®}35 (polyoxyethylene lauryl ether) as extraction buffer. Sample content of semaglutide aldehyde adducts was determined using a RP-UHPLC method. The UHPLC method was based on gradient elution on a C18 column. The eluent system was 0.09 M (NH₄)₂HPO₄ (pH 3.2), acetonitrile and 2-propanol in water with UV detection at 215 nm.

Semaglutide formaldehyde adduct was calculated as % based on the area of the semaglutide formaldehyde adduct peak relative to the area of the semaglutide peak in the chromatogram.

Semaglutide acetaldehyde adduct was calculated as % based on the area of the semaglutide acetaldehyde adduct peak relative to the area of semaglutide peak in the chromatogram.

### Example 1 - Stability of GLP-1 in compositions with and without histidine

Compositions of mixtures between semaglutide and SNAC with and without histidine where prepared according to table 1.1 below. The compositions where prepared using semaglutide or co-spray-dried semaglutide and histidine, each weighed into a container and mixed with SNAC. The co-spray-dried semaglutide and histidine was prepared according to method 1.

**Table 1.1 - Compositions of mixtures with semaglutide and SNAC with and without histidine.**

| | Mixture X (No histidine) | Mixture Y (with histidine, 1:32 molar ratio) |
|---|---|---|
| Semaglutide | 41 mg | 41 mg |
| Histidine | - | 50 mg |
| SNAC | 5000 mg | 5000 mg |

The compositions were stored at 60 °C and 75 % RH in equilibrated desiccators prepared with a saturated NaCl suspension. Prior to storage, one desiccator was additionally added a volume of a 1 mg/mL formaldehyde solution, corresponding to a molar ratio of around 1:1 between formaldehyde and semaglutide.

The levels of semaglutide formaldehyde adduct in the compositions before and after storage were measured according to assay II. The results are presented in table 1.2 below and shows an increase in the levels of semaglutide formaldehyde adduct after 19 days, which is significantly lower for the composition comprising histidine (Mixture Y).

**Table 1.2 - Levels of semaglutide formaldehyde adduct in the mixture compositions as a function of storage time at 60 °C and 75 % RH.**

| With formaldehyde added to desiccator | Storage time | Mixture X (No histidine | Mixture Y (with histidine, 1:32 molar ratio) |
|---|---|---|---|
| - | Start | 0.1 % | 0.1 % |
| No | 19 days | 7.5 % | 1.7 % |
| Yes | 19 days | 94.4 % | 65.5 % |

### Example 2 - Stability of GLP-1 in tablet compositions with and without histidine

A series of tablet compositions comprising semaglutide with and without histidine were prepared according to table 2.1 below. The compositions were prepared with semaglutide or co-spray-dried semaglutide and histidine as described in method 1 and with granules of SNAC and magnesium stearate prepared according to method 2. Pre-mixed semaglutide and histidine were prepared by alternatingly mixing and grinding equal amounts of semaglutide and histidine in a mortar with a pestle.

Granules of SNAC and magnesium stearate for one tablet were weighed onto a weighing pan. Semaglutide, semaglutide and histidine, pre-mixed semaglutide and histidine, or co-spray-dried semaglutide and histidine for one tablet was then weighed on top of the granules in the weighing pan. In all tablets, the molar ratio of semaglutide to histidine was 1:32.

The components were then manually mixed and transferred directly to the tableting die cavity of the instrumented rotary tableting machine (Fette 102i). The mixture in the die cavity was compressed into a round convex tablet with a diameter of 7 mm at a die table rotation speed of 20 rpm and a compression force around 6.5 kN.

**Table 2.1 - Tablet compositions A-D given with the component amounts in mg/tablet.**

| | Tablet A (no histidine) | Tablet B (with histidine ) | Tablet C (with pre-mixed histidine) | Tablet D (with co-spray-dried histidine) |
|---|---|---|---|---|
| Semaglutide | 3 | 3 | 3 | 3 |
| Histidine | - | 3.7 | 3.7 | 3.7 |
| SNAC | 100 | 100 | 100 | 100 |
| Magnesium stearate | 2.6 | 2.6 | 2.6 | 2.6 |

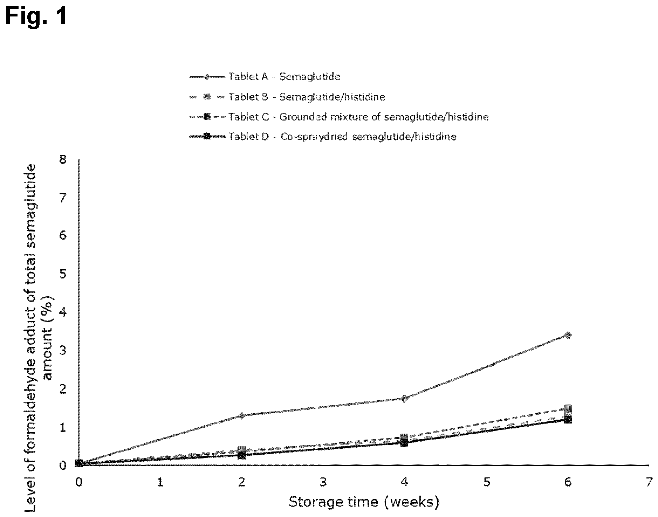

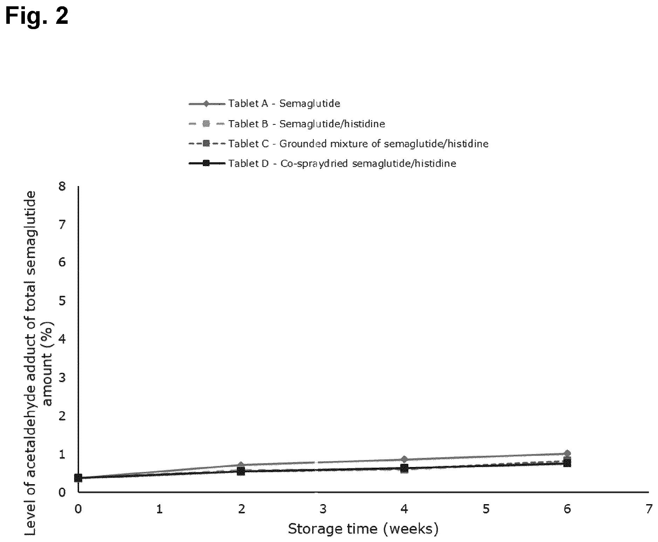

The compositions were stored at 60 °C and 75 % RH in equilibrated desiccators prepared with a saturated NaCl suspension. After storage times of 2, 4, and 6 weeks, tablets were withdrawn from the desiccators and the levels of semaglutide aldehyde adducts were measured according to assay II. The results are included in the tables 2.2 and 2.3 below and in figures 1 and 2 and show an increase in the levels of semaglutide aldehyde adducts over time. The levels of semaglutide aldehyde adducts, and particularly the semaglutide formaldehyde adduct, were significantly lower in the compositions comprising histidine (Tablets B, C, and D).

**Table 2.2 - Levels of semaglutide formaldehyde adduct in the tablet compositions A-D as a function of storage time at 60 °C and 75 %.**

| Storage time | Tablet A (No histidine) | Tablet B (With histidine, 1:32 molar ratio) | Tablet C (pre-mixed with histidine, 1:32 molar ratio) | Tablet D (co-spray-dried with histidine, 1:32 molar ratio) |
|---|---|---|---|---|
| Start | 0.1 % | 0.1 % | 0.1 % | 0.1 % |
| 2 weeks | 1.3 % | 0.4 % | 0.4 % | 0.3 % |
| 4 weeks | 1.8 % | 0.7 % | 0.7 % | 0.6 % |
| 6 weeks | 3.4 % | 1.3 % | 1.5 % | 1.2 % |

**Table 2.3 - Levels of semaglutide acetaldehyde adduct in the tablet compositions A-D as a function of storage time at 60 °C and 75 %.**

| Storage time | Tablet A (No histidine) | Tablet B (With histidine, 1:32 molar ratio) | Tablet C (pre-mixed with histidine, 1:32 molar ratio) | Tablet D (co-spray-dried with histidine, 1:32 molar ratio) |
|---|---|---|---|---|
| Start | 0.4 % | 0.4 % | 0.4 % | 0.4 % |
| 2 weeks | 0.7 % | 0.6 % | 0.5 % | 0.6 % |
| 4 weeks | 0.9 % | 0.6 % | 0.6 % | 0.6 % |
| 6 weeks | 1.0% | 0.8 % | 0.8 % | 0.8 % |

### Example 3 - Stability of GLP-1 in tablet compositions with different amounts of histidine

A further series of tablet compositions comprising semaglutide with and without histidine where prepared according to table 3.1 below. The compositions were prepared with semaglutide or co-spray-dried semaglutide and histidine according to method 1.

For tablet compositions I-IV, granules of SNAC and magnesium stearate were prepared according to method 2 and granules for one tablet were weighed onto a weighing pan. Semaglutide or co-spray-dried semaglutide and histidine for one tablet was afterwards weighed on top of the granules in the weighing pan. The components were then manually mixed and transferred directly to the tableting die cavity of the instrumented rotary tableting machine (Fette 102i). The mixture in the die cavity was compressed into a round convex tablet with a diameter of 6.5 mm at a die table rotation speed of 20 rpm and a compression force around 7.5 kN.

For tablet compositions V-VIII, granules of SNAC, microcrystalline cellulose, and magnesium stearate and granules of microcrystalline cellulose and povidone were generally prepared as described in WO2013/139695 but with slightly different equipment and parameter settings. The granules of microcrystalline cellulose and povidone were pre-mixed with magnesium stearate for 30 min at 25 rpm using a Turbula mixer. Granules of SNAC, microcrystalline cellulose, and magnesium stearate for one tablet were weighed onto a weighing pan. On top of that, granules of microcrystalline cellulose and povidone pre-mixed with magnesium stearate for one tablet was weighed. Semaglutide or co-spray-dried semaglutide and histidine for one tablet was lastly weighed on top of the other components in the weighing pan. The components were then manually mixed and transferred directly to the tableting die cavity of the instrumented rotary tableting machine (Fette 102i). The mixture in the die cavity was compressed into an oval convex tablet with dimensions of 7.5 x 13 mm at a die table rotation speed of 20 rpm and a compression force around 9 kN.

**Table 3.1 - Tablet compositions I-VIII.**

| The compositions are provided as the total amounts of each ingredient in mg/tablet. Tablet I and Tablet V were prepared without histidine while tablets II-IV and VI-VIII included different amounts of histidine, wherein the molar ratio of semglutide to histidine ranges from 1:1 to 1:30 as indicated. | | | | | | |
|---|---|---|---|---|---|---|
| For magnesium stearate, the number in parenthesis is the amount of extra-granular magnesium stearate. For microcrystalline cellulose, the numbers in parenthesis provide the amounts in granules with SNAC and magnesium stearate and in granules with povidone, respectively. | | | | | | |
| | Semaglutide | Histidine | SNAC | Magnesium stearate | Microcrystalline cellulose | Povidone |
| Tablet I (no histidine) | 3 | - | 100 | 2.6 | - | - |
| Tablet II (with histidine, 1:30 molar ratio) | 3 | 3.4 | 100 | 2.6 | - | - |
| Tablet III (with histidine, 1:7.5 molar ratio) | 3 | 0.85 | 100 | 2.6 | - | - |
| Tablet IV (with histidine, 1:1 molar ratio) | 3 | 0.1 | 100 | 2.6 | - | - |
| Tablet V (no histidine) | 3 | - | 300 | 9.7 (2) | 80 (57/23) | 8 |
| Tablet VI (with histidine, 1:30 molar ratio) | 3 | 3.4 | 300 | 9.7 (2) | 80 (57/23) | 8 |
| Tablet VII (with histidine, 1:7.5 molar ratio) | 3 | 0.85 | 300 | 9.7 (2) | 80 (57/23) | 8 |
| Tablet VIII (with histidine, 1:1 molar ratio) | 3 | 0.1 | 300 | 9.7 (2) | 80 (57/23) | 8 |

The tablet compositions I and V were stored at 60 °C, 40 °C and 30 °C at 75 % RH in an equilibrated desiccator prepared with a saturated NaCl suspension. Likewise, the tablet compositions II-IV and VI-VIII were stored at 60 °C, 40 °C and 30 °C at 75 % RH but in another equilibrated desiccator also prepared with a saturated NaCl suspension. Prior to storage, volumes of a 0.012 % w/w formaldehyde solution were added to the desiccators corresponding to a molar ratio of around 1:20 between formaldehyde and semaglutide.

After a storage time of at least 1 month, tablets were withdrawn from the desiccators and the levels of hydrophobic impurities groups 1 and 2 were measured according to assay I and the levels of semaglutide aldehyde adducts were measured according to assay III. Another volume of a 0.012 % w/w formaldehyde solution was added to the desiccator corresponding to a molar ratio of around 1:20 between formaldehyde and semaglutide inside the desiccator before the remaining tablets in the desiccator were stored further.

The results from assay I are included in the tables 3.2 and 3.3 below and show an increase in the levels of hydrophobic impurities groups 1 and 2 over time. The levels of hydrophobic impurities groups 1 and 2 were significantly lower in the compositions comprising histidine (Tablets II-IV and VI-VIII). Furthermore, it is particular surprising that the levels of hydrophobic impurities group 1 were reduced significantly even at the lowest molar ratio of 1:1 between semaglutide and histidine and that the reduction becomes relatively less when increasing the molar ratio of histidine to semaglutide.

**Table 3.2 - Levels of hydrophobic impurities group 1 in the tablet compositions I-VIII as a function of storage time at 60 °C and 75 % RH.**

| | Start | 1 month |
|---|---|---|
| Tablet I (no histidine) | 2.7 % | 38.7 % |
| Tablet II (with histidine, 1:30 molar ratio) | 2.7 % | 24.1 % |
| Tablet III (with histidine, 1:7.5 molar ratio) | 2.7 % | 26.7 % |
| Tablet IV (with histidine, 1:1 molar ratio) | 2.7 % | 24.8 % |
| Tablet V (no histidine) | 2.7 % | 53.9 % |
| Tablet VI (with histidine, 1:30 molar ratio) | 2.5 % | 31.9 % |
| Tablet VII (with histidine, 1:7.5 molar ratio) | 2.6 % | 38.0 % |
| Tablet VIII (with histidine, 1:1 molar ratio) | 2.7 % | 44.5 % |

**Table 3.3 - Levels of hydrophobic impurities group 2 in the tablet compositions I-VIII as a function of storage time at 60 °C and 75 % RH.**

| | Start | 1 month |
|---|---|---|
| Tablet I (no histidine) | 0.0% | 9.4 % |
| Tablet II (with histidine, 1:30 molar ratio) | 0.0% | 4.1 % |
| Tablet III (with histidine, 1:7.5 molar ratio) | 0.0 % | 4.6 % |
| Tablet IV (with histidine, 1:1 molar ratio) | 0.0% | 6.0% |
| Tablet V (no histidine) | 0.0% | 33.0 % |
| Tablet VI (with histidine, 1:30 molar ratio) | 0.1 % | 10.8 % |
| Tablet VII (with histidine, 1:7.5 molar ratio) | 0.1 % | 14.8 % |
| Tablet VIII (with histidine, 1:1 molar ratio) | 0.1 % | 32.6 % |

The results from assay III are included in the tables 3.4.1-3.4.3 and 3.5.1-3.5-3 below and show an increase in the levels of semaglutide aldehyde adducts over time. The levels of semaglutide aldehyde adducts were significantly lower in the compositions comprising histidine (Tablets II-IV and VI-VIII). Furthermore, it is particular surprising that the levels of semaglutide aldehyde adducts were reduced significantly, i.e. more than 50 % even with the lowest amount of histidine, representing a molar ratio of 1:1 between semaglutide and histidine and that the further reductions obtained when increasing the molar ratio of histidine to semaglutide are relatively small.

**Table 3.4.1 - Levels of semaglutide formaldehyde adduct in the tablet compositions I-VIII as a function of storage time at 60 °C and 75 % RH.**

| | Start | 1 month | 3 months |
|---|---|---|---|
| Tablet I (no histidine) | 0.8 % | 18.5 % | 76.6 |
| Tablet II (with histidine, 1:30 molar ratio) | 0.8 % | 3.4 % | 18.5 % |
| Tablet III (with histidine, 1:7.5 molar ratio) | 0.8 % | 5.2 % | 40.2 % |
| Tablet IV (with histidine, 1:1 molar ratio) | 0.8 % | 7.0% | 31.7 % |
| Tablet V (no histidine) | 0.8 % | 129.8 % | 152.3 % |
| Tablet VI (with histidine, 1:30 molar ratio) | 0.8 % | 4.7 % | 24.9 % |
| Tablet VII (with histidine, 1:7.5 molar ratio) | 0.8 % | 8.5 % | 44.8 % |
| Tablet VIII (with histidine, 1:1 molar ratio) | 0.8 % | 36.2 % | 106.9 % |

**Table 3.4.2 - Levels of semaglutide formaldehyde adduct in the tablet compositions I-VIII as a function of storage time at 40 °C and 75 % RH.**

| | Start | 3 months | 6 months |
|---|---|---|---|
| Tablet I (no histidine) | 0.8 % | 8.5 % | 14.8 % |
| Tablet II (with histidine, 1:30 molar ratio) | 0.8 % | 3.4 % | 5.9 % |
| Tablet III (with histidine, 1:7.5 molar ratio) | 0.8 % | 4.5 % | 8.4 % |
| Tablet IV (with histidine, 1:1 molar ratio) | 0.8 % | 4.5 % | 9.2 % |
| Tablet V (no histidine) | 0.8 % | 25.4 % | 53.3 % |
| Tablet VI (with histidine, 1:30 molar ratio) | 0.8 % | 3.0% | 5.5 % |
| Tablet VII (with histidine, 1:7.5 molar ratio) | 0.8 % | 5.0 % | 8.3% |
| Tablet VIII (with histidine, 1:1 molar ratio) | 0.8 % | 9.6 % | 16.3 % |

**Table 3.4.3 - Levels of semaglutide formaldehyde adduct in the tablet compositions I-VIII as a function of storage time at 30 °C and 75 % RH.**

| | Start | 3 months | 6 months |
|---|---|---|---|
| Tablet I (no histidine) | 0.8 % | 4.3 % | 7.7 % |
| Tablet II (with histidine, 1:30 molar ratio) | 0.8 % | 2.1 % | 3.9 % |
| Tablet III (with histidine, 1:7.5 molar ratio) | 0.8 % | 2.4 % | 5.9 % |
| Tablet IV (with histidine, 1:1 molar ratio) | 0.8 % | 2.2 % | 5.4 % |
| Tablet V (no histidine) | 0.8 % | 7.7 % | 18.7 % |
| Tablet VI (with histidine, 1:30 molar ratio) | 0.8 % | 3.0 % | 3.7 % |
| Tablet VII (with histidine, 1:7.5 molar ratio) | 0.8 % | 2.7 % | 4.8 % |
| Tablet VIII (with histidine, 1:1 molar ratio) | 0.8 % | 3.9 % | 7.0 % |

**Table 3.5.1 - Levels of semaglutide acetaldehyde adduct in the tablet compositions I-VIII as a function of storage time at 60 °C and 75 % RH.**

| | Start | 1 month | 3 months |
|---|---|---|---|
| Tablet I (no histidine) | 0.8 % | 31.9 % | 127.5 % |
| Tablet II (with histidine, 1:30 molar ratio) | 0.9 % | 11.9 % | 46.6 % |
| Tablet III (with histidine, 1:7.5 molar ratio) | 0.8 % | 13.6 % | 66.8 % |
| Tablet IV (with histidine, 1:1 molar ratio) | 0.8 % | 13.1 % | 64.4 % |
| Tablet V (no histidine) | 0.8 % | 141.7 % | 176.4 % |
| Tablet VI (with histidine, 1:30 molar ratio) | 0.9 % | 20.4 % | 69.9 % |
| Tablet VII (with histidine, 1:7.5 molar ratio) | 0.9 % | 28.1 % | 87.0 % |
| Tablet VIII (with histidine, 1:1 molar ratio) | 0.8 % | 53.7 % | 117.7 % |

**Table 3.5.2 - Levels of semaglutide acetaldehyde adduct in the tablet compositions I-VIII as a function of storage time at 40 °C and 75 % RH.**

| | Start | 3 months | 6 months |
|---|---|---|---|
| Tablet I (no histidine) | 0.8 % | 7.6 % | 10.5 % |
| Tablet II (with histidine, 1:30 molar ratio) | 0.9 % | 5.4 % | 10.3 % |
| Tablet III (with histidine, 1:7.5 molar ratio) | 0.8 % | 5.3 % | 10.3 % |
| Tablet IV (with histidine, 1:1 molar ratio) | 0.8 % | 4.8 % | 10.3 % |
| Tablet V (no histidine) | 0.8 % | 23.4 % | 36.3 % |
| Tablet VI (with histidine, 1:30 molar ratio) | 0.9 % | 5.8 % | 12.8 % |
| Tablet VII (with histidine, 1:7.5 molar ratio) | 0.9 % | 7.4 % | 15.8 % |
| Tablet VIII (with histidine, 1:1 molar ratio) | 0.8 % | 10.5 % | 20.4 % |

**Table 3.5.3 - Levels of semaglutide acetaldehyde adduct in the tablet compositions I-VIII as a function of storage time at 30 °C and 75 % RH.**

| | Start | 3 months | 6 months |
|---|---|---|---|
| Tablet I (no histidine) | 0.8 % | 2.7 % | 4.0 % |
| Tablet II (with histidine, 1:30 molar ratio) | 0.9 % | 2.3 % | 3.2 % |
| Tablet III (with histidine, 1:7.5 molar ratio) | 0.8 % | 2.1 % | 3.2 % |
| Tablet IV (with histidine, 1:1 molar ratio) | 0.8 % | 1.9 % | 2.8 % |
| Tablet V (no histidine) | 0.8 % | 4.7 % | 9.3 % |
| Tablet VI (with histidine, 1:30 molar ratio) | 0.9 % | 2.3 % | 3.2 % |
| Tablet VII (with histidine, 1:7.5 molar ratio) | 0.9 % | 2.5 % | 4.1 % |
| Tablet VIII (with histidine, 1:1 molar ratio) | 0.8 % | 3.1 % | 4.7 % |

## Claims

1. A solid pharmaceutical composition comprising
i) semaglutide,
ii) a salt of N-(8-(2-hydroxybenzoyl)amino)caprylic acid (NAC) and
iii) histidine.

2. The composition according to any of the previous claims, wherein the molar ratio of histidine to semaglutide is at least 0.5.

3. The composition according to any of the previous claims, wherein the composition further comprises one or more pharmaceutical excipients.

4. The composition according to any of the previous claims, wherein the composition comprises a lubricant, such as magnesium stearate.

5. The composition according to any of the previous claims, wherein the composition comprises 0.25-5 % w/w, such as 1-4 % w/w lubricant of the amount of NAC.

6. The composition according to any of the previous claims, wherein the salt of N-(8-(2-hydroxybenzoyl)amino)caprylic acid (NAC) is sodium N-(8-(2-hydroxybenzoyl)amino)caprylate (SNAC).

7. The composition according to any of the previous claims wherein the composition is a pharmaceutical composition for oral administration.

8. The composition according to any of the previous claims, wherein the composition is a tablet.

9. The composition according to any of the above claims comprising or consisting of:
i) semaglutide,
ii) a salt of N-(8-(2-hydroxybenzoyl)amino)caprylic acid (NAC), such as sodium N-(8-(2-hydroxybenzoyl)amino)caprylate (SNAC),
iii) histidine,
iv) a lubricant and
v) optionally further pharmaceutical excipient(s).

10. The composition according to any of the above claims, wherein a dose unit comprises
i) 0.5-100 mg, such as 0.5-50 mg Semaglutide,
ii) 50-750 mg salt of N-(8-(2-hydroxybenzoyl)amino)caprylic acid (NAC), such sodium N-(8-(2-hydroxybenzoyl)amino)caprylate (SNAC),
iii) 0.01-200 mg histidine,
iv) 1-15 mg lubricant,
v) optionally 1-20 mg binder, such as povidone,
vi) optionally 30-500 mg disintegrant, such as nicotinamide, and
vii) optionally 20-100 mg filler, such as MCC.

11. The composition according to any of the previous claims, wherein the composition is a pharmaceutical composition for use in a method of treating diabetes and/or obesity.

12. A method for producing a solid pharmaceutical composition comprising the steps of;
i) obtaining a blend comprising semaglutide and histidine,
ii) co-processing the blend of i) and
iii) preparing said solid pharmaceutical composition using the product of ii).

13. A method for producing a solid pharmaceutical composition comprising the steps of;
i) obtaining a solution comprising semaglutide and histidine,
ii) spray-drying the solution of i),
iii) preparing said solid pharmaceutical composition using the product of ii).

14. A method for producing a solid pharmaceutical composition comprising the steps of
i) obtaining a blend comprising a salt of NAC and a lubricant,
ii) granulating the blend of i) by dry-granulation,
iii) admixing the granules of ii) with semaglutide and histidine and optionally any further excipients and
iv) preparing said solid pharmaceutical composition using the mixture of iii).

15. A method for producing a solid pharmaceutical composition comprising the steps of
i) obtaining a blend comprising a salt of NAC and a lubricant,
ii) granulating the blend of i) by dry-granulation,
iii) obtaining a solution comprising semaglutide and histidine,
iv) spray-drying the solution of iii),
v) admixing the granules of ii) with the product of iii) and optionally any further excipients and
vi) preparing said solid pharmaceutical composition using the mixture of v).

## Patentansprüche

1. Pharmazeutische Feststoffzusammensetzung, umfassend
i) Semaglutid,
ii) ein Salz von N-(8-(2-Hydroxybenzoyl)amino)caprylsäure (NAC) und
iii) Histidin.

2. Zusammensetzung nach einem der vorhergehenden Ansprüche, wobei das Molverhältnis von Histidin zu Semaglutid mindestens 0,5 beträgt.

3. Zusammensetzung nach einem der vorhergehenden Ansprüche, wobei die Zusammensetzung ferner einen oder mehrere pharmazeutische Hilfsstoffe umfasst.

4. Zusammensetzung nach einem der vorhergehenden Ansprüche, wobei die Zusammensetzung ein Schmiermittel, wie z. B. Magnesiumstearat, umfasst.

5. Zusammensetzung nach einem der vorhergehenden Ansprüche, wobei die Zusammensetzung 0,25-5 Gew.-%, wie z. B. 1-4 Gew.-%, Schmiermittel bezüglich der Menge an NAC umfasst.

6. Zusammensetzung nach einem der vorhergehenden Ansprüche, wobei es sich bei dem Salz von N-(8-(2-Hydroxybenzoyl)amino)caprylsäure (NAC) um Natrium-N-(8-(2-hydroxybenzoyl)amino)caprylat (SNAC) handelt.

7. Zusammensetzung nach einem der vorhergehenden Ansprüche, wobei die Zusammensetzung eine pharmazeutische Zusammensetzung zur oralen Verabreichung ist.

8. Zusammensetzung nach einem der vorhergehenden Ansprüche, wobei es sich bei der Zusammensetzung um eine Tablette handelt.

9. Zusammensetzung nach einem der vorhergehenden Ansprüche, die Folgendes umfasst oder daraus besteht:
i) Semaglutid,
ii) ein Salz von N-(8-(2-Hydroxybenzoyl)amino)caprylsäure (NAC), wie z. B. Natrium-N-(8-(2-hydroxybenzoyl)amino)caprylat (SNAC),
iii) Histidin,
iv) ein Schmiermittel und
v) gegebenenfalls weitere pharmazeutische Hilfsstoffe.

10. Zusammensetzung nach einem der vorhergehenden Ansprüche, wobei eine Dosiseinheit Folgendes umfasst
i) 0,5-100 mg, wie z. B. 0,5-50 mg, Semaglutid,
ii) 50-750 mg Salz von N-(8-(2-Hydroxybenzoyl)amino)caprylsäure (NAC), wie z. B. Natrium-N-(8-(2-hydroxybenzoyl)amino)caprylat (SNAC),
iii) 0,01-200 mg Histidin,
iv) 1-15 mg Schmiermittel,
v) gegebenenfalls 1-20 mg Bindemittel, wie z. B. Povidon,
vi) gegebenenfalls 30-500 mg Sprengmittel, wie z. B. Nicotinamid, und
vii) gegebenenfalls 20-100 mg Füllstoff, wie z. B. MCC.

11. Zusammensetzung nach einem der vorhergehenden Ansprüche, wobei die Zusammensetzung eine pharmazeutische Zusammensetzung zur Verwendung bei einem Verfahren zur Behandlung von Diabetes und/oder Adipositas ist.

12. Verfahren zur Herstellung einer pharmazeutischen Feststoffzusammensetzung, umfassend die Schritte:
i) Erhalten einer Mischung, die Semaglutid und Histidin umfasst,
ii) gemeinsames Verarbeiten der Mischung von i) und
iii) Herstellen der pharmazeutischen Feststoffzusammensetzung unter Verwendung des Produkts von ii).

13. Verfahren zur Herstellung einer pharmazeutischen Feststoffzusammensetzung, umfassend die Schritte:
i) Erhalten einer Lösung, die Semaglutid und Histidin umfasst,
ii) Sprühtrocknen der Lösung von i),
iii) Herstellen der pharmazeutischen Feststoffzusammensetzung unter Verwendung des Produkts von ii).

14. Verfahren zur Herstellung einer pharmazeutischen Feststoffzusammensetzung, umfassend die Schritte:
i) Erhalten einer Mischung, die ein Salz von NAC und ein Schmiermittel umfasst,
ii) Granulieren der Mischung von i) durch Trockengranulierung,
iii) Beimischen von Semaglutid und Histidin und gegebenenfalls weiteren Hilfsstoffen zu dem Granulat von ii) und
iv) Herstellen der pharmazeutischen Feststoffzusammensetzung unter Verwendung des Gemischs von iii).

15. Verfahren zur Herstellung einer pharmazeutischen Feststoffzusammensetzung, umfassend die Schritte:
i) Erhalten einer Mischung, die ein Salz von NAC und ein Schmiermittel umfasst,
ii) Granulieren der Mischung von i) durch Trockengranulierung,
iii) Erhalten einer Lösung, die Semaglutid und Histidin umfasst,
iv) Sprühtrocknen der Lösung von iii),
v) Beimischen des Produkts von iii) und gegebenenfalls weiterer Hilfsstoffe zu dem Granulat von ii) und
vi) Herstellen der pharmazeutischen Feststoffzusammensetzung unter Verwendung des Gemischs von v).

## Revendications

1. Composition pharmaceutique solide comprenant
i) sémaglutide,
ii) un sel d'acide N-(8-(2-hydroxybenzoyl)amino)caprylique (NAC) et
iii) histidine.

2. Composition selon l'une quelconque des revendications précédentes, dans laquelle le rapport molaire de l'histidine au sémaglutide est d'au moins 0,5.

3. Composition selon l'une quelconque des revendications précédentes, dans laquelle la composition comprend en outre un ou plusieurs excipients pharmaceutiquement.

4. Composition selon l'une quelconque des revendications précédentes, dans laquelle la composition comprend un lubrifiant tel qu'un stéarate de magnésium.

5. Composition selon l'une quelconque des revendications précédentes, dans laquelle la composition comprend de 0,25 à 5 % en poids, tel que 1 à 4 % en poids de lubrifiant de la quantité de NAC.

6. Composition selon l'une quelconque des revendications précédentes, dans laquelle l'acide N-(8-(2-hydroxybenzoyl)amino)caprilyque (NAC) est le sel sodique d'acide N-(8-(2-hydroxybenzoyl)amino)caprylique (SNAC).

7. Composition selon l'une quelconque des revendications précédentes, dans laquelle la composition est une composition pharmaceutique pour administration orale.

8. Composition selon l'une quelconque des revendications précédentes, dans laquelle la composition est un comprimé.

9. Composition selon l'une quelconque des revendications précédentes, comprenant ou constituée de :
i) sémaglutide,
ii) un sel de l'acide N-(8-(2-hydroxybenzoyl)amino)caprylique (NAC), tel que le N-(8-(2-hydroxybenzoyl)amino)caprylate de sodium (SNAC),
iii) histidine,
iv) un lubrifiant et
v) facultativement d'un autre ou d'autres excipients pharmaceutiques.

10. Composition selon l'une quelconque des revendications précédentes, dans laquelle une dose unitaire comprend
i) 0,5 à 100 mg, tel que 0,5 à 50 mg de sémaglutide,
ii) 50 à 750 mg de sel d'acide N-(8-(2-hydroxybenzoyl)amino)caprylique (NAC), tel que le N-(8-(2-hydroxybenzoyl)amino)caprylate de sodium (SNAC),
iii) 0,01 à 200 mg d'histidine,
iv) 1 à 15 mg de lubrifiant,
v) facultativement 1 à 20 mg de liant, tel que la povidone,
vi) facultativement 30 à 500 mg de désintégrant, tel que le nicotinamide, et
vii) éventuellement 20 à 100 mg de charge, telle que MCC.

11. Composition selon l'une quelconque des revendications précédentes, dans laquelle la composition est une composition pharmaceutique pour une utilisation dans un procédé de traitement du diabète et/ou de l'obésité.

12. Procédé de production d'une composition pharmaceutique solide comprenant les étapes suivantes ;
i) l'obtention d'un mélange comprenant du sémaglutide et de l'histidine,
ii) le co-traitement du mélange de i) et
iii) la préparation de ladite composition pharmaceutique solide en utilisant le produit de ii).

13. Procédé de production d'une composition pharmaceutique solide comprenant les étapes suivantes ;
i) l'obtention d'une solution comprenant du sémaglutide et de l'histidine,
ii) le séchage par pulvérisation la solution de i),
iii) la préparation de ladite composition pharmaceutique solide en utilisant le produit de ii).

14. Procédé de production d'une composition pharmaceutique solide comprenant les étapes de
i) l'obtention d'un mélange comprenant un sel de NAC et un lubrifiant,
ii) la granulation du mélange de i) par granulation à sec,
iii) l'incorporation des granules de ii) avec du sémaglutide et de l'histidine et facultativement d'autres excipients et
iv) la préparation de ladite composition pharmaceutique solide en utilisant le mélange de iii).

15. Procédé de production d'une composition pharmaceutique solide comprenant les étapes de
i) l'obtention d'un mélange comprenant un sel de NAC et un lubrifiant,
ii) la granulation du mélange de i) par granulation à sec,
iii) l'obtention d'une solution comprenant du sémaglutide et de l'histidine,
iv) le séchage par pulvérisation la solution de iii),
v) l'incorporation des granules de ii) avec le produit de iii) et éventuellement d'autres excipients et
vi) la préparation de ladite composition pharmaceutique solide en utilisant le mélange de v).
